# EUROPEAN PATENT APPLICATION

(11) **EP 4 046 653 A1**
(43) Date of publication of application: **24.08.2022**
(21) Application number: 21382125.9
(22) Date of filing: 17.02.2021
(51) Int. Cl.: A61K 39/12, A61P 31/12, C07K 14/165, G01N 33/53

(54) **IMMUNOGENIC POLYPEPTIDES AND USES THEREOF**

(71) Applicant: Consejo Superior De Investigaciones Científicas, 28006 Madrid (ES)
(72) Inventor: RODRÍGUEZ AGUIRRE, José Francisco, E-28049 Cantoblanco, Madrid (ES); RODRÍGUEZ AGUIRRE, Mª Dolores, E-28049 Cantoblanco, Madrid (ES); SANTIAGO HERNÁNDEZ, César Augusto, E-28049 Cantoblanco, Madrid (ES); VARAS RIOS, Antonio Jesús, E-28049 Cantoblanco, Madrid (ES); ALMAZÁN TORAL, Fernando, E-28049 Cantoblanco, Madrid (ES); RODRÍGUEZ FERNÁNDEZ-ALBA, Juan Ramón, E-28049 Cantoblanco, Madrid (ES)
(74) Representative: ABG Intellectual Property Law, S.L.

(57) **Abstract**

The present invention relates to new polypeptides, more specifically to immunogenic polypeptides derived from protein Spike of SARS-CoV-2. It also relates to the use of the said polypeptides in eliciting an immune response, preventing or treating a disease caused by the infection by SARS-CoV-2 and detecting the presence of neutralizing antibodies against SARS-CoV-2.

## Description

### FIELD OF THE INVENTION

The present invention is comprised within the field of biomedicine. It specifically relates to polypeptides and polynucleotides, and the use thereof in the prevention or treatment of a disease caused by SARS-CoV-2 infection.

### BACKGROUND OF INVENTION

Coronaviruses (CoV) are enveloped viruses with a positive-stranded RNA genome. An outbreak of severe acute respiratory syndrome (SARS) occurred in 2002 infecting over 8000 people with about 10 percent fatality rate. A novel coronavirus was identified as the agent causing SARS and named SARS-CoV. Another coronavirus, the Middle East respiratory syndrome coronavirus (MERS-CoV) was identified in 2012, which infected over 2000 people in 27 countries with about 35 percent fatality rate. In December 2019, a novel beta-coronavirus related to MERS-CoV and SARS-CoV and designated SARS-CoV-2 or 2019-nCoV was identified in Wuhan, China. WHO has declared SARS-CoV-2 a public health emergency of international concern (PHEIC), and since March 2020, characterized the situation as a global pandemic. The disease caused by SARS-CoV-2 is designated COVID-19 and severely affects the health of millions of patients globally, causing an ever-increasing number of deaths around the world. Although the native protein structure of SARS-CoV-2 is known, it displays a poor immunogenicity. For these reasons, there is an urgent need of immunogenic compounds and vaccines against SARS-CoV-2. Further, the impact of SARS-CoV-2 on economy and healthcare systems raised the interest in providing methods for distinguishing among non-immunized healthy, immunized healthy and infected subjects. Thus, there is a need in the art for the identification of adequate methods for the early detection of infections caused by SARS-CoV-2 as well as for the treatment of the diseases caused by said infection.

### SUMMARY OF THE INVENTION

In a first aspect the invention relates to a polypeptide comprising
(i) a receptor binding domain (RBD) region derived from protein S of SARS-CoV-2 as defined in SEQ ID NO:1 or a functionally equivalent and immunologically active variant thereof, and
(ii) a histidine-rich region ,
wherein the polypeptide does not comprise a region comprising a second receptor binding domain (RBD) derived from protein S of SARS-CoV-2 as defined in SEQ ID NO:1 or of functionally equivalent and immunologically active variant thereof.

In another aspect, the invention relates to a dimer polypeptide which is formed by two monomer polypeptides, wherein each monomer polypeptide is as defined in the preceding paragraph and wherein the monomer polypeptides are not connected by a covalent bond between their respective distal N- or C-terminal amino acids.

In another aspect, the invention relates to a dimeric polypeptide, wherein each monomer polypeptide comprises a receptor binding domain (RBD) region derived from protein S of SARS-CoV-2 as defined in SEQ ID NO:1 or a functionally equivalent and immunologically active variant thereof, and wherein the monomer polypeptides are not connected by a covalent bond between their respective distal N- or C-terminal amino acids.

In another aspect, the invention relates to a method of producing a dimeric polypeptide as defined in the preceding paragraph comprising the steps of:
(i) expressing in a suitable expression system a polynucleotide encoding a polypeptide comprising
   a. a receptor binding domain (RBD) region derived from protein S of SARS-CoV-2 as defined in SEQ ID NO:1 or a functionally equivalent and immunologically active variant thereof,
   b. a histidine-rich region, and
   c. a protease target site between regions (a) and (b),
   wherein the expression is carried out under conditions adequate for allowing the dimerization of said polypeptides, so that dimer polypeptides are produced and;
(ii) contacting the dimer polypeptides obtained in step (i) with a protease which is capable of recognizing and cleaving the target site found in the monomers, thereby producing dimer polypeptides devoid of the histidine-rich regions.

In another aspect, the invention relates to a polypeptide comprising
(i) a receptor binding domain (RBD) region derived from protein S of SARS-CoV-2 as defined in SEQ ID NO:1 or a functionally equivalent and immunologically active variant thereof, and
(ii) a trimerization domain.

In another aspect, the invention relates to trimeric polypeptide which is formed by three monomer polypeptides, wherein each monomer polypeptide is as defined in the preceding paragraph.

In another aspect the invention relates to a polynucleotide encoding the polypeptide of the invention.

In another aspect, the invention relates to a vector comprising the polynucleotide encoding the polypeptide of the invention.

In another aspect, the invention also relates to a host cell comprising the polynucleotide encoding the polypeptide of the invention or the vector comprising the polynucleotide encoding the polypeptide of the invention.

In another aspect, the invention relates to the polypeptide, the oligomer, the polynucleotide or the vector of the invention for use in the prevention or treatment of a disease caused by the infection of SARS-CoV-2

In another aspect, the invention relates to an immunogenic composition or vaccine comprising the polypeptide, the polynucleotide or the vector of the invention and a pharmaceutically acceptable carrier.

In another aspect the invention also relates to a method for producing a polypeptide comprising a receptor binding domain (RBD) derived from protein S of SARS-CoV-2 or a functionally equivalent and immunologically active variant thereof, comprising the steps of
(i) expressing in suitable expression system a polynucleotide of the invention so as to produce the protein encoded by the polynucleotide and
(ii) recovering the recombinant protein from the supernatant.

In another aspect the invention relates to method for the detection of the presence of antibodies specific for the protein S of SARS-CoV-2 in a sample comprising the steps of
(i) contacting the sample with the polypeptide or the oligomer of the invention,
(ii) detecting the formation of a complex between antibodies specific for the SARS-CoV-2 S protein present in the sample and the polypeptide or oligomer of step (i).

In another aspect, the invention relates to a method for detecting whether a subject has suffered an infection by SARS-CoV-2 comprising the detection of antibodies against the SARS-CoV-2 spike protein in a sample of the subject by the method of the invention.

In another aspect, the invention relates to a method for producing a population of antibodies against SARS-CoV-2, said method comprising the steps of:
i) administering the polypeptides or oligomers according to the invention into a subject so that an immune response against said polypeptides or oligomers is elicited in said subject and;
ii) isolating from said subject antibodies, antibody fragments and/or antibody-producing cells recognizing the polypeptides or oligomers according to the invention.

### BRIEF DESCRIPTION OF THE FIGURES

**Figure 1****-** Analysis by chromatographic profile and SDS-PAGE of the different purified RBD proteins.
**Figure 2****.-** RBD1 and RBD2 are recognized similarly in ELISA by COVID-19 positive human sera.
**Figure 3****.-** Anti RBD-antibody responses are higher in mice immunized with RBD1 vs RBD2.
**Figure 4****.-** Anti RBD-antibody responses are higher in mice immunized with RBD1 or RBD3 vs RBD2.

### DETAILED DESCRIPTION OF THE INVENTION

The present invention relates to the provision of new polypeptides and polynucleotides for the prevention or treatment of diseases related to a SARS-CoV-2 infection as well as new methods for detecting antibodies against SARS-CoV-2.

### Polypeptides according to the invention

The authors of the invention have found that polypeptides derived from the Spike protein of SARS-CoV-2 have immunogenic activities. Hence, the invention relates to polypeptides (hereinafter "first polypeptides of the invention" or "dimer-forming polypeptides of the invention") with the capacity to act as immunogenic agents and serve as components of vaccines. As shown in the examples, the polypeptides of the invention comprising a receptor binding domain (RBD) region derived from protein S of SARS-CoV-2 as defined in SEQ ID NO:1 can efficiently be expressed in cell culture and subsequently purified and isolated in the form of dimers. These polypeptides showed similar antigenic presentation in ELISA when analyzed head-to-head with a battery of anti-SARS-CoV-2 positive human sera. Notably, the examples confirmed that high humoral immune responses against RBD are induced in test animals after immunization with the polypeptides of the invention.

Thus, in a first aspect the invention relates to a polypeptide (herein after polypeptides of the invention or dimer-forming polypeptides of the invention) comprising
(i) a receptor binding domain (RBD) region derived from protein S of SARS-CoV-2 as defined in SEQ ID NO:1 or a functionally equivalent and immunologically active variant thereof, and
(ii) a histidine-rich region,
wherein the polypeptide does not comprise a region comprising a second receptor binding domain (RBD) region derived from protein S of SARS-CoV-2 as defined in SEQ ID NO:1 or of functionally equivalent and immunologically active variant thereof.

The terms "polypeptide" and "protein" are used interchangeably. The term "protein" refers to a sequence of amino acids composed of the naturally occurring amino acids as well as derivatives thereof. The naturally occurring amino acids are well known in the art and are described in standard text books of biochemistry. Within the amino acid sequence the amino acids are connected by peptide bonds. Further, the two ends of the amino acid sequence are referred to as the carboxyl terminus (C-terminus) and the amino terminus (N-terminus). The term "protein" encompasses essentially purified proteins or protein preparations comprising other proteins in addition. Further, the term also relates to protein fragments. Moreover, it includes chemically modified proteins. Such modifications may be artificial modifications or naturally occurring modifications such as phosphorylation, glycosylation, myristylation and the like.

The polypeptide may be provided in any form and at any degree of purification, from tissues, fluids or cells comprising said polypeptide in an endogenous form, more preferably cells overexpressing the polypeptide, crude or enriched lysates of such cells, to purified and/or isolated polypeptide which may be essentially pure. In one embodiment, the polypeptide is a native polypeptide, wherein the term "native polypeptide", as used herein, refers to a folded polypeptide, more preferably to a folded polypeptide purified from cells, more preferably from prokaryotic or eukaryotic, preferably mammalian cells. A glycosylated form of the polypeptide may be used.

The term "receptor binding domain (RBD) region as defined in SEQ ID NO:1" as used herein refers to any polypeptide derived from protein S of SARS-CoV-2 that has the amino acid sequence of the receptor binding domain (RBD) region of the spike protein and that consists, consist essentially of or comprises SEQ ID NO: 1 but wherein it does not comprise the complete protein S of SARS-CoV-2, the transmembrane domain of the S protein of SARS-CoV-2 or the cytosolic tail of the of the S protein of SARS-CoV-2. The term also refers to polypeptides or proteins comprising, consisting essentially of or consisting of the amino acid sequence SEQ ID NO:1, which is located at positions 319-541 of the Spike protein of the WuhanSHu-1 isolate of the SARS-CoV-2 virus identified as UniProtKB - P0DTC2 (SPIKE_SARS2), Version 5, Swiss-Prot Release 2020_06; pubDate Wed, 2 December 2020 in the UniProt database. The genome of the SARS-CoV-2 virus provided in the NCBI database under accession No. MN908947.3. Further, "RBD polypeptides derived from protein S of SARS-CoV-2" or "RBD proteins" refer to those proteins encoded by nucleic acid molecules, which hybridize under conditions of high stringency to the nucleic acid strand complementary to the coding sequence of the receptor binding domain (RBD) region of the spike protein of SARS-CoV-2.

"Functionally equivalent variant" is understood to mean all those polypeptides derived from the sequence SEQ ID NO:1 by modification, substitution, insertion and/or deletion of one or more amino acids whenever the function is substantially maintained. According to the present invention, the relevant function to be substantially maintained is the ability of the polypeptide to dimerize. The ability of a polypeptide or variant to dimerize can be assessed experimentally, for example, by SDS-PAGE in non-reducing conditions followed by Coomassie-blue staining or by its chromatographic purification profile, as shown in the examples of the present application. This method allows the determination of the relative abundance and molecular masses of dimeric forms with respect to monomeric forms, which can be visualized and compared.

Without wishing to be bound by theory, the authors have found that (i) the presence of a histidine-rich region in the RBD results in the formation of dimers and (ii) the dimers show an enhanced immunogenicity and production of neutralizing antibodies as shown by the Examples.

In a preferred embodiment, the "functionally equivalent variant" displays an ability to dimerize when connected to an histidine rich region which is of at least 40%, at least 50%, at least 60%, at least 70%, at least 75%, at least 80%, at least 85%, at least 90%, at least 91 %, at least 92%, at least 93%, at least 94%, at least 95%, at least 96%, at least 97%, at least 98%, at least 99%, or at least 100% of the ability shown by the sequence SEQ ID NO:1.

Preferably, "variants" of sequence SEQ ID NO:1 are (i) polypeptides in which one or more amino acid residues are substituted by a conserved or non-conserved amino acid residue (preferably a preserved amino acid residue) and such substituted amino acid may be coded or not by the genetic code, (ii) polypeptides in which there is one or more modified amino acid residues, for example, residues modified by substituent bonding, (iii) polypeptides resulting from alternative processing of a similar mRNA, (iv) polypeptides fragments and/or (v) polypeptides resulting from the fusion of sequence SEQ ID NO:1 with itself or of the variants defined above in (i) to (iii) with another polypeptide, such as a secretory leader sequence or a signal peptide (for example the signal peptide of the *Autographa californica* nuclear polyhedrosis virus (AcNPV) protein gp67 or the signal peptide of the Spike protein (GenBank: MN908947.3) of SARS-CoV-2), a sequence being used for purification (for example, His tag), for detection (for example, Sv5 epitope tag) or for delivery to a specific target cell. The fragments include polypeptides generated through proteolytic cut (including multisite proteolysis) of an original sequence. The functionally equivalent variant may be the result of a post-translationally or chemically modification. Such variants will be apparent to those skilled in the art.

The "variants" of sequence SEQ ID NO:1 may be both natural and artificial. The expression "natural variant" relates to all those variants of SEQ ID NO:1 which appear naturally in other species, i.e. the orthologues of SEQ ID NO:1. Examples of variants of SEQ ID NO:1 include but are not restricted to N501S N501H V503F T478I F490Y, Y453F, V445A, A475S, N439K, A475V, F490S, G446V, S477I, S477N, G476S, T478R, N501Y, E484K, N437S, S477T, S494P. These variants may display higher binding affinity to the receptor ACE2 than SEQ ID NO:1.

A "functionally equivalent variant of sequence SEQ ID NO:1 can be an amino acid sequence derived from sequence SEQ ID NO:1 comprising the addition, substitution or modification of one or more amino acid residues. By way of illustration, equivalent variants of the sequences SEQ ID NO:1 include sequences comprising the addition of 1 amino acid, 2 amino acids, 3 amino acids, 4 amino acids, 5 amino acids, 10 amino acids, 15 amino acids, 20 amino acids, 25 amino acids, 30 amino acids, 35 amino acids, 40 amino acids, 45 amino acids, 50 amino acids, 60 amino acids, 70 amino acids, 80 amino acids, 90 amino acids, 100 amino acids, 150 amino acids, 200 amino acids, at least 500 amino acids, at least 1000 amino acids or more at the amino terminus of the sequence SEQ ID NO:1 and/or comprising the addition of 1 amino acid, 2 amino acids, 3 amino acids, 4 amino acids, 5 amino acids, 10 amino acids, 1 1 amino acids, 12 amino acids, 13 amino acids, 14 amino acids, 15 amino acids, 20 amino acids, 25 amino acids, 30 amino acids, 35 amino acids, 40 amino acids, 45 amino acids, 50 amino acids, 60 amino acids, 70 amino acids, 80 amino acids, 90 amino acids, 100 amino acids, 150 amino acids, 200 amino acids, at least 500 amino acids, at least 1000 amino acids or more at the carboxy terminus of sequence SEQ ID NO:1, and maintaining at least 50%, at least 60%, at least 70%, at least 75%, at least 80%, at least 85%, at least 90%, at least 91 %, at least 92%, at least 93%, at least 94%, at least 95%, at least 96%, at least 97%, at least 98%, at least 99%, or at least 100% of the activity of the sequence SEQ ID NO:1.

The activity or function of the sequence SEQ ID NO:1 and its functionally equivalent variants can also be determined by assaying the immunogenic activity of the polypeptides by a method shown in the examples of the present application.

"Functionally equivalent variants" of the sequence SEQ ID NO:1 also include amino acid sequences with a sequence identity of at least 70%, at least 75%, at least 80%, at least 85%, at least 90%, at least 91 %, at least 92%, at least 93%, at least 94%, at least 95%, at least 96%, at least 97%, at least 98%, or at least 99% with the sequence SEQ ID NO:1.

In a particular embodiment the "functionally equivalent variant" of the polypeptide of the invention has a sequence which shows at least 80% identity with the sequence of SEQ ID NO:1. The terms "identity", "identical" or "percent identity" in the context of two or more amino acid or nucleotide sequences, refer to two or more sequences or subsequences that are the same or have a specified percentage of amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences. Publicly available software programs can be used to align sequences. Appropriate parameters for maximal alignment by particular alignment software can be determined by one skilled in the art. In certain embodiments, the default parameters of the alignment software are used. In certain embodiments, the percentage identity "X" of a first amino acid sequence to a second amino acid sequence is calculated as 100 x (Y/Z), where Y is the number of amino acid residues scored as identical matches in the alignment of the first and second sequences (as aligned by visual inspection or a particular sequence alignment program) and Z is the total number of residues in the second sequence. If the second sequence is longer than the first sequence, then the global alignment taken the entirety of both sequences into consideration is used, therefore all letters and null in each sequence must be aligned. In this case, the same formula as above can be used but using as Z value the length of the region wherein the first and second sequence overlaps, said region having a length which is substantially the same as the length of the first sequence.

As a non-limiting example, whether any particular polypeptide has a certain percentage sequence identity (e.g., is at least 80% identical, at least 85% identical, at least 90% identical, and in some embodiments, at least 95%, 96%, 97%, 98%, or 99% identical) to a reference sequence. For instance, 95% identical to a reference sequence according to the present invention, the parameters are set such that the percentage of identity is calculated over the full length of the reference amino acid sequence and that gaps in homology of up to 5% of the total number of nucleotides in the reference sequence are allowed.

In some embodiments, two amino acid sequences are substantially identical, meaning they have at least 70%, at least 75%, at least 80%, at least 85%, at least 90%, and in some embodiments at least 95%, 96%, 97%, 98%, 99% amino acid residue identity, when compared and aligned for maximum correspondence, as measured using a sequence comparison algorithm or by visual inspection. Identity can exist over a region of the sequences that is at least about 10, about 20, about 40-60 residues in length or any integral value there between, and can be over a longer region than 60-80 residues, for example, at least about 90-100 residues, and in some embodiments, the sequences are substantially identical over the full length of the sequences being compared.

The term "immunologically active variant" refers to a polypeptide that comprises at least one antigen, which elicits an immunological response in the host to which the immunogenic polypeptide is administered. Such immunological response may be a cellular and/or antibody-mediated immune response to the immunogenic polypeptide of the invention. Preferably, the immunogenic polypeptide induces an immune response and, more preferably, confers protective immunity against one or more of the clinical signs of a SARS-CoV-2 infection. The host is also described as "subject". Preferably, any of the hosts or subjects described or mentioned herein without limitation human or mammal. Usually, an "immunological response" includes but is not limited to one or more of the following effects: the production or activation of antibodies, B cells, helper T cells, suppressor T cells, and/or cytotoxic T cells and/or gamma-delta T cells, directed specifically to an antigen or antigens included in the immunogenic composition of the invention. Preferably, the host will display either a protective immunological response or a therapeutically response.

The term "immunologically active variant" is understood to mean all those immunologically active polypeptides derived from the sequence SEQ ID NO:1 by modification, substitution, insertion and/or deletion of one or more amino acids, whenever the function of eliciting an immunological response in the host to which the immunologically active polypeptide is administered is substantially maintained. The term "immunologically active variant" as used herein also refers to a variant of a polypeptide that retains substantially equivalent ability to induce an immune response in a subject as the reference polypeptide. The term "immunologically active variant" is well understood in the art and is further defined in detail herein. Immunologically functional equivalents may increase the antigenicity of a polypeptide, maintain the same level of antigenicity of the reference polypeptide, or decrease the antigenicity of a polypeptide only slightly so that it maintains its usefulness as an antigen in an immunogenic composition.

The term "immunologically active variant", as used herein, refers to variants which are able to generate an immune response which differs from the immune response generated with the native region by no more than 5 %, no more than 10 %, no more than 15 %, no more than 20 %, no more than 25 %, no more than 30 %, no more than 35 %, no more than least 40 %, no more than 45 %, no more than 50%, no more than 55 %, no more than 60 %, no more than 65 %, no more than 70 %, no more than 75 %, no more than 80 %, no more than 85 %, no more than 90 % or no more than 95 %. For the purposes of the present invention, a polypeptide that is useful as an antigen in an immunogenic composition (i.e. has sufficient "immunogenic activity") can be identified by any method commonly known in the art for measuring the ability of a given polypeptide to trigger the generation of antibodies specific for said polypeptide when administered to a host organism. The ability of a given variant of the polypeptide of the invention to be an immunologically equivalent variant may be determined using standard neutralization assays, wherein the suspected variant is inoculated into a test animal and the resulting antibodies are tested for their ability to neutralize the infection of susceptible cells by SAR-CoV-2 virus. Any known and/or conventional method for screening and identifying and obtaining the neutralizing antibodies of the invention are contemplated by the present invention. In certain examples, a cell line or pseudovirus assay is used as a neutralization assay. Such assays are well known in the art.

Accordingly, "immunologically functional equivalent" of a receptor binding domain (RBD) derived from protein S of SARS-CoV-2 as defined in SEQ ID NO:1 shows a degree of identity with respect to the amino acid sequence of the corresponding sequence in SEQ ID NO:1 of at least 50%, at least 55%, at least 60%, at least 65%, at least 70%, at least 75%, at least 80%, at least 85%, at least 90%, at least 95%, at least 96%, at least 97%, at least 98% or at least 99% provided the immunogenic activity of the protein is maintained. Preferably, the identity exists over a region that is at least about 25 amino acids or nucleotides in length, or more preferably over a region that is 50-100 amino acids or nucleotides in length. An indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. The degree of identity between two peptides can be determined using computer algorithms and methods, which are widely known by the persons skilled in the art.

As an example of modifications contemplated to be within the scope of the present invention, certain amino acids may be substituted for other amino acids in a polypeptide structure without appreciable loss of interactive binding capacity of the structure such as, for example, the epitope of an antigen that is recognized and bound by an antibody. Since it is the interactive capacity and nature of a polypeptide that defines its biological (e.g. immunological) functional activity, certain amino acid sequence substitutions can be made in an amino acid sequence (or its underlying DNA coding sequence) and nevertheless obtain a polypeptide with comparable properties. Various changes may be made to the amino acid sequences of the antigens of the present invention without appreciable loss of immunogenic activity.

It is understood in the art that in order to make functionally equivalent amino acid substitutions, the hydropathic index of amino acids may be considered. The importance of the hydropathic amino acid index in conferring interactive biological function on a polypeptide is generally understood in the art. It is known that certain amino acids may be substituted for other amino acids having a similar hydropathic index or score and still retain a similar biological activity. In making changes based upon the hydropathic index, the substitution of amino acids whose hydropathic indices are within plus or minus 2 is preferred, those which are within plus or minus 1 are particularly preferred, and those within plus or minus 0.5 are even more particularly preferred. Each amino acid has been assigned a hydropathic index on the basis of its hydrophobicity and charge characteristics; these are: isoleucine (+4.5), valine (+4.2), leucine (+3.8), phenylalanine (+2.8), cysteine/cystine (+2.5), methionine (+1.9), alanine (+1.8), glycine (-0.4), 5 threonine (-0.7), serine (-0.8), tryptophan (-0.9), tyrosine (-1.3), proline (-1.6), histidine (-3.2), glutamate (-3.5), glutamine (-3.5), aspartate (-3.5), asparagine (-3.5), lysine (-3.9) and arginine (-4.5). It also is understood in the art that the substitution of similar amino acids can be made effectively on the basis of hydrophilicity; particularly where the immunologically functional equivalent polypeptide thereby created is intended for use in immunological embodiments, as in certain embodiments of the present invention. The greatest local average hydrophilicity of a protein, as governed by the hydrophilicity of its adjacent amino acids, correlates with its immunogenicity and antigenicity. In making changes based upon similar hydrophilicity values, the substitution of amino acids whose hydrophilicity values are within plus or minus 2 is preferred, those which are within plus or minus 1 are particularly preferred, and those within plus or minus 0.5 are even more particularly preferred. The following hydrophilicity values have been assigned to amino acid residues: arginine (+3.0), lysine (+3.0), aspartate (+3.0 plus or minus 1), glutamate (+3.0 plus or minus 1), serine (+0.3), asparagine 20 (+0.2), glutamine (+0.2), glycine (0), threonine (-0.4), proline (-0.5 plus or minus 1), alanine (-0.5), histidine (-0.5), cysteine (-1.0), methionine (-1.3), valine (-1.5), leucine (-1.8), isoleucine (-1.8), tyrosine (-2.3), phenylalanine (-2.5) and tryptophan (-3.4).

Different types of amino acid sequences may be added to the core polypeptide to attain different objectives such as, for example, facilitating their handling or monitoring their expression. In one embodiment, the addition of these peptide sequences to the core polypeptide may does not affect their immunogenic capabilities. For instance, a di-, tri-, tetra-, penta-, hexa-, hepta-, octa-, nona-, deca-histidine tag sequence can be added to all the polypeptides of the present invention to facilitate their purification. In another embodiment, however, the present invention contemplates that the position with respect to the core polypeptide, and the resulting length or number of histidine residues may surprisingly enhance the immunogenicity of the polypeptides of the invention.

In one embodiment, the receptor binding domain (RBD) region derived from protein S of SARS-CoV-2 as defined in SEQ ID NO:1 or the functionally equivalent and immunologically active variant thereof refers to a region which comprises the receptor binding domain (RBD) region of protein S region but which does not contain the complete sequence of protein S of SARS-CoV2.

In another embodiment, the receptor binding domain (RBD) region derived from protein S of SARS-CoV-2 as defined in SEQ ID NO:1 or the functionally equivalent and immunologically active variant thereof consists essentially or consists of the amino acid sequence of the receptor binding domain (RBD) of protein S.

In a further embodiment, the receptor binding domain (RBD) region derived from protein S of SARS-CoV-2 as defined in SEQ ID NO:1 or a functionally equivalent and immunologically active variant thereof comprises at the most 1200 amino acid residues, 1100 amino acids, 1000 amino acids, 900 amino acids, 800 amino acids, 700 amino acids, 600 amino acids, 500 amino acids, 400 amino acids, 300 amino acids, 290 amino acids, 280 amino acids, 270 amino acids, 260 amino acids or 250 amino acids of the sequence of protein S of SARS-CoV2 as defined in the UniProt database under accession number P0DTC2.

In some embodiments, the receptor binding domain (RBD) region derived from protein S of SARS-CoV-2 as defined in SEQ ID NO:1 or a functionally equivalent and immunologically active variant thereof substantially lack or completely lack any part or the totality of the transmembrane region or the cytosolic tail of the protein S of SARS-CoV-2 as defined in SEQ ID NO:1.

In one embodiment, the a receptor binding domain (RBD) region derived from protein S of SARS-CoV-2 as defined in SEQ ID NO:1 comprises, consists essentially or consists of the region formed by amino acids 319 to 541 of the sequence of protein S of SARS-CoV2 as defined in the UniProt database under accession number P0DTC2.

The term "histidine-rich region" or "polyhistidine tail" or "his-tag" is understood as an amino acid sequence comprising histidine amino acids in a proportion of at least 50%, at least 60%, at least 70%, at least 75%, at least 80%, at least 85%, at least 90%, and in some embodiments at least 95%, 96%, 97%, 98%, 99% histidine residues of the total amino acids within the histidine-rich region. The histidine-rich region may comprise, consists essentially or consists of at least 2 amino acids, at least 3 amino acids, at least 4 amino acids, at least 5 amino acids, at least 6 amino acids, at least 7 amino acids, at least 8 amino acids, at least 9 amino acids, at least 10 amino acids, at least 11 amino acids, at least 12 amino acids, at least 13 amino acids, at least 14 amino acids, at least 15 amino acids, at least 16 amino acids, at least 17 amino acids, at least 18 amino acids, at least 19 amino acids, at least 20 amino acids, at least 21 amino acids, at least 22 amino acids, at least 23 amino acids, at least 24 amino acids or at least 25 amino acids.

In some embodiments, the histidine-rich region comprises, consists essentially of or consists of an amino acid sequence having at least 2 histidine amino acids, at least 3 histidine amino acids, at least 4 histidine amino acids, at least 5 histidine amino acids, at least 6 histidine amino acids, at least 7 histidine amino acids, at least 8 histidine amino acids, at least 9 histidine amino acids, at least 10 histidine amino acids, at least 11 histidine amino acids, at least 12 histidine amino acids, at least 13 histidine amino acids, at least 14 histidine amino acids, at least 15 histidine amino acids, at least 16 histidine amino acids, at least 17 histidine amino acids, at least 18 histidine amino acids, at least 19 histidine amino acids, at least 20 histidine amino acids, at least 21 histidine amino acids, at least 22 histidine amino acids, at least 23 histidine amino acids, at least 24 histidine amino acids or at least 25 histidine amino acids. In one embodiment, the histidine-rich region comprises, consists essentially of or consists of 9 consecutive histidine amino acids. In one embodiment, the histidine-rich region comprises, consists essentially of or consists of 6 consecutive histidine amino acids

The term "histidine-rich region" may also refer to any amino acid sequence with the ability to preferential binding to metal chelating resins which typically include a transition metal such as Ni or Co.

The term "polypeptide does not comprise a region comprising a second receptor binding domain (RBD) derived from protein S of SARS-CoV-2 as defined in SEQ ID NO:1 or of functionally equivalent and immunologically active variant thereof" is understood as the absence in the amino acid sequence of the polypeptide according to the invention of a further amino acid sequence comprising a further receptor binding domain (RBD) derived from protein S of SARS-CoV-2 as defined in SEQ ID NO:1 or of functionally equivalent and immunologically active variant thereof.

The term "N-terminally" or "N-terminus" or "amino-terminus" or "NH₂-terminus" or "N-terminal end" or "amine-terminus" is understood as the start of an amino acid sequence of a protein or polypeptide referring to the free amine group (-NH₂) located at one end of a polypeptide. The term "C-terminus" or "carboxyl-terminus" or "carboxy-terminus" or "C-terminal tail" or "C-terminal end" or "COOH-terminus" is the end of an amino acid chain of a protein or polypeptide terminated by a free carboxyl group (-COOH). When the protein or polypeptide is translated from messenger RNA, it is created from N-terminus to C-terminus. The convention for writing polypeptide sequences is to represent the N-terminus on the left side and C-terminus on the right side of the amino acid sequence. The amino acid sequence reads from N- to C-terminus.

In a preferred embodiment, the histidine-rich region according to the invention is located N-terminally with respect to the receptor binding domain (RBD) region or to the functionally equivalent and immunologically active variant thereof, and comprises 9 histidine amino acids.

In one embodiment, the polypeptide according to the invention further comprises a protease target site between the region (i) and (ii).

The term "protease target site", as used herein, refers to a polypeptide sequence which can be recognized and processed by a protease when the target site and the protease are contacted under suitable conditions. Suitable protease target sites for use in the present invention and the corresponding protease include:
- The LVPRGS target site as defined in SEQ ID NO:13, which is cleaved by thrombin,
- The ENLYFQG/S target site as defined in SEQ ID NO:14 or SEQ ID NO:15 which is cleaved by the TEV protein from Tobacco Etch Virus,
- The IE/DGR target site as defined in SEQ ID NO:16 or SEQ ID NO:17 which is processed by factor Xa,
- The LEVLFQGP target site as defined in SEQ ID NO:18 which is processed by the PreScission Protease (rPSP) from human rhinovirus (HRV 3C)).

In another preferred embodiment the polypeptide according to the invention is defined in SEQ ID NO:2.

The term "defined in SEQ ID NO:2" refers to polypeptides or proteins comprising the amino acid sequence SEQ ID NO:2:
In a preferred embodiment, the histidine-rich region according to the invention is located C-terminally with respect to the receptor binding domain (RBD) region or a functionally equivalent and immunologically active variant thereof, and comprises 6 histidine amino acids.

In another preferred embodiment, the polypeptide according to the invention is defined in SEQ ID NO:3.

The term "defined in SEQ ID NO:3" refers to polypeptides or proteins comprising the amino acid sequence SEQ ID NO:3.

The authors have found that polypeptides derived from protein S of SARS-CoV-2 may be fused to a trimerization domain, that these fusion proteins are capable of forming trimers and that the resulting trimers induce a strong humoral immune response against RBD. Further, the results also showed that RBD epitopes are better exposed in polypeptides derived from protein S of SARS-CoV-2 combined with a trimerization domain.

Accordingly, in another aspect, the invention relates to a polypeptide (hereinafter "second polypeptide of the invention" or "trimer-forming polypeptide of the invention" comprising
(i) a receptor binding domain (RBD) region derived from protein S of SARS-CoV-2 as defined in SEQ ID NO:1 or a functionally equivalent and immunologically active variant thereof, and
(ii) a trimerization domain.

The terms "RBD" and "protein S from SARS-CoV-2" have been described in the context of the first polypeptides of the invention and are equally applicable to the trimers of the invention.

The term "functionally equivalent variant" has been described in detail in the context of first polypeptides of the invention and applies equally to the polypeptide comprising the trimerization domain with the proviso that the variant is characterized in that it preserves the ability of forming a trimer when fused to the trimerization domain. In a preferred embodiment, the "functionally equivalent variant" of the trimer-forming polypeptide displays an ability to trimerize when connected to trimerization domain which is of at least 40%, at least 50%, at least 60%, at least 70%, at least 75%, at least 80%, at least 85%, at least 90%, at least 91 %, at least 92%, at least 93%, at least 94%, at least 95%, at least 96%, at least 97%, at least 98%, at least 99%, or at least 100% of the ability shown by the sequence SEQ ID NO:1. Suitable methods for determining whether a RBD variant polypeptide preserves its ability to trimerize when fused to a trimerization domain are as explained above in the context of the RBD containing the histidine-rich region, i.e. by polyacrylamide gel electrophoresis under non-denaturing conditions followed by staining using any dye suitable for protein staining or by Western blot using an anti-RBD-specific antibody.

The term "immunologically active variant" has been explained above in the context of first polypeptide of the invention and applies equally to the polypeptide comprising the trimerization domain.

In one embodiment, the a receptor binding domain (RBD) region derived from protein S of SARS-CoV-2 as defined in SEQ ID NO:1 comprises, consists essentially or consists of the region formed by amino acids 329 to 541 of the sequence of protein S of SARS-CoV2 as defined in the UniProt database under accession number P0DTC2. In one embodiment, the a receptor binding domain (RBD) region derived from protein S of SARS-CoV-2 is as defined in SEQ ID NO:19.

The term "trimerization domain" is understood as an amino acid sequence whose function is to promote folding and trimerization of monomer polypeptides. Examples of suitable trimerization domains include without restriction, the T4 bacteriophage foldon trimerization domain, the trimerization domain of collagen XV or the trimerization domain of collagen XVIII.

In one embodiment, the receptor binding domain (RBD) region derived from protein S of SARS-CoV-2 as defined in SEQ ID NO:1 or a functionally equivalent and immunologically active variant thereof refers to the amino acid sequence of the receptor binding domain (RBD) of protein S which consists of amino acids 319 to 541 of the SARS-CoV2 S protein as provided in the UniProtKB database under accession number P0DTC2 and wherein the complete sequence of protein S of SARS-CoV2 is excluded.

In another embodiment, the receptor binding domain (RBD) region derived from protein S of SARS-CoV-2 as defined in SEQ ID NO:1 or a functionally equivalent and immunologically active variant thereof consists essentially or consists of the amino acid sequence of the receptor binding domain (RBD) of protein S.

In a further embodiment, a receptor binding domain (RBD) region region derived from protein S of SARS-CoV-2 as defined in SEQ ID NO:1 or a functionally equivalent and immunologically active variant thereof and which forms part of the trimer-forming polypeptide according to the invention contains at the most 1200 amino acid residues, 1100 amino acids, 1000 amino acids, 900 amino acids, 800 amino acids, 700 amino acids, 600 amino acids, 500 amino acids, 400 amino acids, 300 amino acids, 290 amino acids, 280 amino acids, 270 amino acids, 260 amino acids or 250 amino acids of the protein S of SARS-CoV2.

In some embodiments, the receptor binding domain (RBD) region derived from protein S of SARS-CoV-2 as defined in SEQ ID NO:1 or a functionally equivalent and immunologically active variant thereof substantially lacks or completely lacks any part of or the totality of the transmembrane region and/or the cytosolic tail of the protein S of SARS-CoV-2.

In some embodiments, the trimerization domain is located N-terminally with respect to region or to the functionally equivalent and immunologically active variant thereof.

In some embodiments, the trimerization domain is located C-terminally with respect to the receptor binding domain (RBD) region or to the functionally equivalent and immunologically active variant thereof.

In a preferred embodiment, the polypeptide of the invention comprises SEQ ID NO:4, in which the RBD region consists of the sequence located at positions 329-541 of the Spike protein of the WuhanSHu-1 isolate of the SARS-CoV-2 virus identified as UniProtKB - P0DTC2 (SPIKE_SARS2), Version 5, Swiss-Prot Release 2020_06; pubDate Wed, 2 December 2020 in the UniProt database.

In a further preferred embodiment, the polypeptide of the invention comprises positions 329-541 of the Spike protein as defined in SEQ ID NO:19.

In another preferred embodiment, the trimerization domain according to the invention comprises SEQ ID NO:5.

The term "trimerization domain comprises SEQ ID NO:5" refers to polypeptides or proteins comprising the amino acid sequence SEQ ID NO:5

In a preferred embodiment, the polypeptide further comprises an affinity tag, preferably a histidine rich region.

As used herein, the term "affinity tag" refers to an amino acid sequence, which has affinity for a specific capture reagent and which can be separated from a pool of polypeptides. The "affinity tag" allows isolation from other components on the basis of its affinity for the binding partner. The affinity tag may form part of a target polypeptide sequence to be purified. Where the affinity tag forms part of the target polypeptide, the tag may remain part of the target polypeptide following purification and not be cleaved therefrom.

Alternatively, the affinity tag may be attached to the target peptide. For example, the affinity tag may be covalently linked to the target polypeptide via an amino acid linker. The amino acid linker may comprise a cleavage site to enable detachment of the affinity tag from the target protein. Selective cleavage sites and methods of cleaving such sequences are well known in the art. The affinity tag may be attached to the target peptide at the C-terminal or the N-terminal end thereof.

As used herein, the term "affinity tag" may refer to an affinity tag consisting of monomeric amino acid units of a single amino acid. Preferably the monomeric units are amino acid residues having affinity for a capture reagent. Examples of monomeric units include but are not restricted to Histidine, Arginine, Cysteine, Phenylalanine, Lysine, Glutamic acid and Aspartic acid residues. Examples of homopolymeric tags include polyHis-tags, polyArg-tags, polyCys-tags, polyPhe-tags, polyLys-tags, polyGlu-tags and polyAsp-tags.

The "affinity tag" may comprise a plurality of combinations in diverse proportions of different monomeric amino acid units, for example, Histidine, Arginine, Cysteine, Phenylalanine, Lysine, Glutamic acid and Aspartic acid residues.

Preferably, the "affinity tag" comprises at least 3 monomeric units of a monomeric unit. More preferably, the "affinity tag" comprises at least 4, 5, 6, 7, 8, 9, 10, 11, 12, 13, 14, 15, 20, 25 or 30 monomeric units of a monomeric unit.

In a preferred embodiment, the affinity tag comprises 9x histidines as defined in SEQ ID NO:12.

As used herein, the term "capture reagent" refers to any agent that preferentially binds to an affinity tag of the present invention. Any suitable capture reagent can be used according to the present invention. The properties of the affinity tag of the invention will necessarily determine the choice of capture reagent used. A person of skill in the art will be aware of the how the binding properties of the affinity tag will dictate the selection of capture reagent.

### Dimers comprising the RBD of the protein S of SARS-CoV-2

The authors of the present invention have found that the expression of the polypeptides of the invention results in the spontaneous assembly of the polypeptides into homodimers in which each of the integrating monomer is a polypeptide according to the invention.

Accordingly, in another aspect, the invention refers to a dimer polypeptide (first type of dimers of the invention) which is formed by two monomer polypeptides, wherein each monomer polypeptide is a polypeptide according to the invention and wherein the monomer polypeptides are not connected by a covalent bond between their respective distal N- or C-terminal amino acids.

The term "homodimer" refers to type of dimer in which the two monomers forming the dimer are identical. The term "dimer" refers to the structure comprising two monomers, for example, two monomer polypeptides, which can be identical in the case of the homodimers or different in the case of heterodimers.

The term "monomer" is understood as a molecule that can react chemically together with other monomer molecules to form a larger polymer chain or three-dimensional network in a process. In the present context, a polypeptide that can covalently or non-covalently react with another polypeptide can be understood as a monomer, for example and without limitation, a polypeptide comprising a receptor binding domain (RBD) derived from protein S of SARS-CoV-2.

The term "oligomer" is understood as a structure comprising two or more monomers and includes both the first type of dimers of the invention, the second type of dimers of the invention and the trimers of the invention which are described below.

The expression "the monomer polypeptides are not connected by a covalent bond between their respective distal N- or C-terminal amino acids", as used herein, indicates that the monomers forming the dimer do not contain any covalent bound between the C-terminus of the first monomer and the N-terminus of the second monomer. Accordingly, in one embodiment, the dimers according to the invention are not single-chain dimers. This does not imply that the monomers within the dimer are not connected by disulfide bonds established between the side chains of the cysteine residues present within each of the monomers. In some embodiments, the dimer of the invention contains at least 1, at least 2, at least 3 or more disulfide bonds. In one embodiment, the dimer contains disulfide bonds between the cysteine residues at position 538 in the respective monomers, wherein the position is indicated with respect to the complete sequence of the SARS-CoV-2 spike protein as defined in the UniProtKB database under accession number P0DTC2 and which corresponds to the cysteine at position 220 in the sequence as defined in SEQ ID NO:1. It will be understood that the number of disulfide bonds connecting the two monomers is not limited by the number of cysteine residues which appear in the native RBD derived from protein S of SARS-CoV-2 as defined in SEQ ID NO:1, as the RBD may be a functionally equivalent variant of the RBD as defined in SEQ ID NO:1 containing additional cysteine residues.

The term "covalent bond" refers to the equal sharing of an electron pair by two atoms, for example, peptide (amide) and disulfide bonds between amino acids, and C-C, C-O, and C-N bonds within amino acids. A non-covalent interaction differs from a covalent bond in that it does not involve the sharing of electrons but rather involves more dispersed variations of electromagnetic interactions between molecules or within a molecule, and include electrostatic interactions, π-effects, van der Waals forces, and hydrophobic effects. According to the invention, monomers can interact with each other through covalent bonds or non-covalent interactions or both.

The term "N-terminal amino acid" refers to the utmost amino acid of the polypeptide chain at its N-terminus. The term "C-terminal amino acid" refers to the utmost amino acid of the polypeptide chain at its C-terminus.

In some embodiments, the first type of dimer according to the invention is formed by monomers in which each of them consist essentially or consist of the RBD derived from protein S of SARS-CoV-2 as defined in SEQ ID NO:1. In some embodiments, the dimers according to the invention are formed by monomers in which each of them substantially lack or completely lack the transmembrane region or the cytosolic tail of the protein S of SARS-CoV-2 as defined in SEQ ID NO:1.

In one embodiment, the first type of dimers of the invention are characterized in that at least one of the monomers forming the dimer further comprises a target site for a protease between the regions (i) and (ii).

In one embodiment, the first type of dimers of the invention are characterized in that they are formed by monomers in which the histidine-rich region is located N-terminally with respect to the receptor binding domain (RBD) region or to the functionally equivalent and immunologically active variant thereof.

In one embodiment, the first type of dimers of the invention are characterized in that they are formed by monomers having the sequence as defined in SEQ ID NO:2.

In one embodiment, the first type of dimers of the invention are characterized in that they are formed by monomers in which the histidine-rich region is located C-terminally with respect to the receptor binding domain (RBD) region or to the functionally equivalent and immunologically active variant thereof.

In one embodiment, the first type of dimers of the invention are characterized in that they are formed by monomers having the sequence as defined in SEQ ID NO:3.

The authors of the present invention have also found out that, when the polypeptide of the present invention in which a protease target site is inserted between the RBD region and the histidine-rich region is expressed and allowed to dimerize and is then treated with a protease which is capable of removing the histidine-rich region, the resulting product is a stable dimer, which indicates that the histidine-rich regions, while important during the formation of the dimers, can then be removed from the dimers without affecting the structural integrity of the dimers.

Accordingly, in another aspect, the invention relates to a dimer polypeptide (the second type of dimer of the invention), wherein each monomer polypeptide comprises the receptor binding domain (RBD) region derived from protein S of SARS-CoV-2 as defined in SEQ ID NO:1 or a functionally equivalent and immunologically active variant thereof, and wherein the monomer polypeptides are not connected by a covalent bond between their respective distal N- or C-terminal amino acids. In one embodiment, the second type of dimer polypeptide is characterized in that none of the monomers contain an histidine rich region.

In some embodiments, the second type of dimer according to the invention is formed by monomers in which each of them consist essentially or consist of the RBD derived from protein S of SARS-CoV-2 as defined in SEQ ID NO:1. In some embodiments, the second type of dimers according to the invention are formed by monomers in which each of them substantially lack or completely lack the transmembrane region or the cytosolic tail of the protein S of SARS-CoV-2 as defined in SEQ ID NO:1.

In one embodiment, the second type of dimers of the invention are characterized in they are obtained by allowing the formation of a dimer formed by the polypeptides of the invention (a first type of dimer) wherein the polypeptides contain a protease target site between region (i) and (ii) followed by treating such a first type of dimer with a protease specific for said target site, thereby resulting in the removal of the histidine-rich regions and the formation of the second type of dimer.

In one embodiment, the second type of dimers of the invention are characterized in that they are formed by monomers in which the histidine-rich region is located N-terminally with respect to the receptor binding domain (RBD) region or to the functionally equivalent and immunologically active variant thereof.

In one embodiment, the second type of dimers of the invention are characterized in that they are formed by monomers in which the histidine-rich region is located C-terminally with respect to the receptor binding domain (RBD) region or to the functionally equivalent and immunologically active variant thereof.

In another aspect, the invention relates to a method of producing a polypeptide in form of a dimer, comprising the steps of:
(i) expressing in a suitable expression system a polynucleotide encoding a polypeptide comprising
   a. a receptor binding domain (RBD) region derived from protein S of SARS-CoV-2 as defined in SEQ ID NO:1 or a functionally equivalent and immunologically active variant thereof,
   b. a histidine-rich region, and
   c. a protease target site between regions (a) and (b),
   wherein the expression is carried out under conditions adequate for allowing the dimerization of said polypeptides, so that dimer polypeptides are produced and;
(ii) contacting the dimer polypeptides obtained in step (i) with a protease which is capable of recognizing and cleaving the target site found in the monomers, thereby producing dimer polypeptides devoid of the histidine-rich regions.

Methods for expressing the polypeptides of the invention are described in detail below and are equally applicable to the step (i) of the method for producing the second type of dimers of the invention.

Step (ii) of the method for producing the second type of dimers of the invention comprises contacting the dimer polypeptides obtained in step (ii) with a protease which is capable of recognizing and cleaving the target site found in the monomers, thereby producing dimer polypeptides devoid of the histidine-rich regions. Conditions adequate for the processing step will depend on the type of protease which is used. Standard conditions for conventional proteases are well known to the person skilled in the art and are not particularly limitative of the scope of the invention.

In some embodiments, the method for obtaining the second type of dimers according to the present invention contain an additional step between steps (i) and (ii) wherein the dimers generated are purified prior to the processing with the protease. In one embodiment, the intermediate purification step is carried out by immobilized metal affinity chromatography (IMAC) based on the affinity of the histidine rich for a metal which is immobilized in a support.

In another embodiment, the dimers obtained after step (ii) are purified and separated from the histidine-rich regions which have been removed during the protease treatment. In one embodiment, the purification step is carried out by size exclusion chromatography.

### Trimeric polypeptides according to the invention

In another aspect, the invention relates to a homotrimer which is formed by three of the timer-forming polypeptides of the invention.

The term "trimer" refers to a structure comprising three monomers, for example, three monomer polypeptides. The term "homotrimer" refers to a structure comprising three identical monomers, for example, three identical monomer polypeptides according to the invention. The ability to trimerize can be assessed experimentally, for example, by SDS-PAGE in non-reducing conditions followed by Coomassie-blue staining, by which the relative abundance and molecular masses of trimeric forms with respect to monomeric forms can be visualized and compared.

### Polynucleotides encoding the polypeptides of the invention, and vectors and host cells containing said polynucleotides according to the invention

In a third aspect the invention relates to a polynucleotide encoding the polypeptide of the invention.

Recombinant techniques may be used to generate the polypeptides of the present invention. To produce a polypeptide of the present invention using recombinant technology, a polynucleotide encoding the polypeptide of the present invention is ligated into a nucleic acid expression vector, which comprises the polynucleotide sequence under the transcriptional control of a cis-regulatory sequence (e.g., promoter sequence) suitable for directing constitutive, species specific, cell specific, tissue specific or inducible transcription of the monomers, dimers or trimers of the present invention in the host cells.

In addition to being synthesizable in host cells, the polypeptides of the present invention can also be synthesized using *in vitro* expression systems. These methods are well known in the art and the components of the system are commercially available.

As used herein, the term "polynucleotide" refers to a polymer composed of a multiplicity of nucleotide units (deoxyribonucleotides or ribonucleotides, or related structural variants or synthetic analogues thereof) linked via phosphodiester bonds (or related structural variants on synthetic analogues thereof). The term polynucleotide includes double or single stranded genomic and cDNA, RNA, any synthetic and genetically manipulated polynucleotide, and both sense and anti-sense polynucleotide (although only sense stands are being disclosed in the present invention). This includes single- and doublestranded molecules, i.e., DNA-DNA, DNA-RNA and RNA-RNA hybrids.

In one embodiment, the polynucleotide of the invention further comprises a sequence encoding a signal peptide in the same open reading frame as the polypeptide.

The term "signal peptide" refers to a short peptide, commonly 5-30 amino acids long, present at the N-terminus of newly synthesized proteins that are directed towards the secretory pathway. These proteins include those that reside either inside certain organelles (for example, the endoplasmic reticulum, Golgi or endosomes), are secreted from the cell, and transmembrane proteins. Signal peptides commonly contain a core sequence which is a long stretch of hydrophobic amino acids that tends to form a single alpha-helix. The signal peptide may begin with a short positively charged stretch of amino acids, which helps to enforce proper topology of the polypeptide during translocation. At the end of the signal peptide there is typically a stretch of amino acids that is recognized and cleaved by signal peptidase. Very large number of signal peptides for insect, yeasts, plant and mammalian cells are known, and are available in databases. For example, http://www.signalpeptide.de lists 2109 confirmed mammalian signal peptides in its database. The invention contemplates the use of any suitable signal peptides selected from the mentioned list.

The term "open reading frame" corresponds to a nucleotide sequence in a DNA or RNA molecule that may potentially encode a polypeptide or a protein. Said open reading frame begins with a start codon (the start codon generally encoding a methionine), followed by a series of codons (each codon encoding an amino acid), and ends with a stop codon (the stop codon not being translated).

In one embodiment, the sequence encoding a signal peptide according to the invention is selected from SEQ ID NO:6 or SEQ ID NO:7, which encode respectively, the polypeptides of SEQ ID NO:10 and 11.

In one preferred embodiment, the polynucleotide according to the invention comprises a sequence optimized for expression in insect cells. DNA sequences must be optimized for its expression in either, insect, yeast, plant or mammalian cells.

In another preferred embodiment, the polynucleotide according to the invention comprises the sequences SEQ ID NO:8 or SEQ ID NO:9.

In a fourth aspect, the invention relates to a vector comprising the polynucleotide encoding the polypeptide of the invention. As it is shown in the examples, the polypeptides according to the invention are efficiently expressed and isolated from the supernatant of insect cell cultures previously hosting the polynucleotide of the invention.

In a fifth aspect, the invention also relates to a host cell comprising the polynucleotide encoding the polypeptide of the invention or the vector comprising the polynucleotide encoding the polypeptide of the invention.

The polynucleotide of the invention can be found isolated as such or forming part of vectors allowing the propagation of said polynucleotides in suitable host cells. Therefore, in another aspect, the invention relates to a vector, hereinafter vector of the invention, encoding the polynucleotide of the invention as described above.

Vectors suitable for the insertion of said polynucleotide are vectors derived from expression vectors in prokaryotes such as pUC18, pUC19, Bluescript and the derivatives thereof, mpl8, mpl9, pBR322, pMB9, ColEI, pCRI, RP4, phages and "shuttle" vectors such as pSA3 and pAT28; expression vectors in yeasts such as vectors of the type of 2 micron plasmids, integration plasmids, YEP vectors, centromere plasmids and the like; expression vectors in insect cells such as vectors of the pAC and pVL; expression vectors in plants such as plBl, pEarleyGate, pAVA, pCAMBIA, pGSA, pGWB, pMDC, pMY, pORE series and the like; and expression vectors in eukaryotic cells, including baculovirus suitable for transfecting insect cells using any commercially available baculovirus system such as pFastBac1. The vectors for eukaryotic cells include preferably viral vectors (adenoviruses, viruses associated to adenoviruses such as retroviruses and, particularly, lentiviruses) as well as non-viral vectors such as pSilencer 4.1-CMV (Ambion), pcDNA3, pcDNA3.1/hyg, pHMCV/Zeo, pCR3.1, pEFI/His, pIND/GS, pRc/HCMV2, pSV40/Zeo2, pTRACER-HCMV, pUB6/V5-His, pVAXI, pZeoSV2, pCI, pSVL and PKSV-10, pBPV-1, pML2d and pTDTI .

The vectors may also comprise a reporter or marker gene which allows identifying those cells that have incorporated the vector after having been put in contact with it.

Useful reporter genes in the context of the present invention include lacZ, luciferase, thymidine kinase, GFP and on the like. Useful marker genes in the context of this invention include, for example, the neomycin resistance gene, conferring resistance to the aminoglycoside G418; the hygromycin phosphotransferase gene, conferring resistance to hygromycin; the ODC gene, conferring resistance to the inhibitor of the ornithine decarboxylase (2-(difluoromethyl)-DL-ornithine (DFMO); the dihydrofolatereductase gene, conferring resistance to methotrexate; the puromycin-N-acetyl transferase gene, conferring resistance to puromycin; the ble gene, conferring resistance to zeocin; the adenosine deaminase gene, conferring resistance to 9-beta-D-xylofuranose adenine; the cytosine deaminase gene, allowing the cells to grow in the presence of N-(phosphonacetyl)-L-aspartate; thymidine kinase, allowing the cells to grow in the presence of aminopterin; the xanthine-guanine phosphoribosyltransferase gene, allowing the cells to grow in the presence of xanthine and the absence of guanine; the trpB gene of E. coli, allowing the cells to grow in the presence of indol instead of tryptophan; the hisD gene of *E.coli,* allowing the cells to use histidinol instead of histidine. The selection gene is incorporated into a plasmid that can additionally include a promoter suitable for the expression of said gene in eukaryotic cells (for example, the CMV or SV40 promoters), an optimized translation initiation site (for example, a site following the so-called Kozak's rules or an IRES), a polyadenylation site such as, for example, the SV40 polyadenylation or phosphoglycerate kinase site, introns such as, for example, the beta-globulin gene intron. Alternatively, it is possible to use a combination of both the reporter gene and the marker gene simultaneously in the same vector.

On the other hand, as the skilled person in the art is aware that the choice of the vector will depend on the host cell in which it will subsequently be introduced. By way of example, the vector in which said polynucleotide is introduced can also be a yeast artificial chromosome (YAC), a bacterial artificial chromosome (BAC) or a PI -derived artificial chromosome (PAC). The characteristics of the YAC, BAC and PAC are known by the person skilled in the art. The vector of the invention can be obtained by conventional methods known by persons skilled in the art.

The polynucleotide of the invention can be introduced into the host cell in vivo as naked DNA plasmids, but also using vectors by methods known in the art, including but not limited to transfection, electroporation (e.g. transcutaneous electroporation), microinjection, transduction, cell fusion, DEAE dextran, calcium phosphate precipitation, use of a gene gun, or use of a DNA vector transporter. Methods for formulating and administering naked DNA to mammalian muscle tissue are also known. Other molecules are also useful for facilitating transfection of a nucleic acid in vivo, such as cationic oligopeptides, peptides derived from DNA binding proteins, or cationic polymers.

Another well-known method that can be used to introduce polynucleotides into host cells is particle bombardment (aka biolistic transformation). Biolistic transformation is commonly accomplished in one of several ways. One common method involves propelling inert or biologically active particles at cells.

Alternatively, the vector can be introduced *in vivo* by lipofection. The use of cationic lipids can promote encapsulation of negatively charged nucleic acids, and also promote fusion with negatively charged cell membranes. Particularly useful lipid compounds and compositions for transfer of nucleic acids have been described.

Thus, in another aspect, the invention relates to a host cell, hereinafter also cell of the invention, comprising the polynucleotide of the invention or a vector of the invention. The cells can be obtained by conventional methods known by persons skilled in the art.

The term "host cell", as used herein, refers to a cell into which a nucleic acid of the invention, such as a polynucleotide or a vector according to the invention, has been introduced and is capable of expressing the polypeptides of the invention as monomers, dimers or trimers or a combination thereof. The terms "host cell" and "recombinant host cell" are used interchangeably herein. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein. The term includes any cultivatable cell that can be modified by the introduction of heterologous DNA. Preferably, a host cell is one in which the polynucleotide of the invention can be stably expressed, post-translationally modified, localized to the appropriate subcellular compartment, and made to engage the appropriate transcription machinery. The choice of an appropriate host cell will also be influenced by the choice of detection signal. For example, reporter constructs, as described above, can provide a selectable or screenable trait upon activation or inhibition of gene transcription in response to a transcriptional regulatory protein; in order to achieve optimal selection or screening, the host cell phenotype will be considered. A host cell of the present invention includes prokaryotic cells and eukaryotic cells. Prokaryotes include gram-negative or gram-positive bacteria, for example, *E. coli* or *Bacilli.* It is to be understood that prokaryotic cells will be used, preferably, for the propagation of the transcription control sequence comprising polynucleotides or the vector of the present invention. Suitable prokaryotic host cells for transformation include, for example, *E. coli, Bacillus subtilis, Salmonella typhimurium,* and various other species within the genera *Pseudomonas, Streptomyces,* and *Staphylococcus.* Eukaryotic cells include, but are not limited to, yeast cells, plant cells, fungal cells, insect cells (e.g., baculovirus), mammalian cells, and the cells of parasitic organisms, e.g., trypanosomes. As used herein, yeast includes not only yeast in a strict taxonomic sense, i.e., unicellular organisms, but also yeast-like multicellular fungi of filamentous fungi. Exemplary species include *Kluyverei lactis, Schizosaccharomyces pombe,* and *Ustilaqo maydis,* with *Saccharomyces cerevisiae* being preferred. Other yeasts which can be used in practicing the present invention are *Neurospora crassa, Aspergillus niger, Aspergillus nidulans, Pichia pastoris, Candida tropicalis,* and *Hansenula polymorpha.* Mammalian host cell culture systems include established cell lines such as COS cells, L cells, 3T3 cells, Chinese hamster ovary (CHO) cells, embryonic stem cells, with BHK, HeK or HeLa, insect cells, for example but not restricted to, BTI-Tn-5B1-4 (Thermo Fisher Scientific) being preferred. Eukaryotic cells are, preferably, used to for recombinant gene expression by applying the transcription control sequence or the expression vector of the present invention.

### Use of the polypeptides, dimers, trimers, polynucleotides and vectors of the invention in the prevention or treatment of a disease caused by SARS-CoV-2 infection

As it is shown in the examples of the present application, the authors of the invention have found out that the polypeptides, oligomers (dimers and trimers) of the invention are able to elicit an immune response in a subject, being therefore useful tools for preventing or treating subjects against SARS-Co-V-2 infections; thereby, the various clinical symptoms associated to SARS-Co-V-2 infection can be prevented, ameliorated or eliminated. The examples demonstrate that immunization with the polypeptide of the invention induces anti-RBD antibodies with a neutralizing capacity against SARS-CoV-2 comparable to a standard purified neutralizing monoclonal antibody against SARS-CoV-2.

Thus, in another aspect, the invention relates to the polypeptide, the oligomer, the polynucleotide or the vector according to the invention for use in the prevention or treatment of a disease caused by SARS-CoV-2 infection.

The term "treatment", as used herein, comprises any type of therapy, which aims at terminating, preventing, ameliorating and/or reducing the susceptibility to a clinical condition as described herein, e.g. SARS-Co-V-2 infection and related conditions. Thus, "treatment," "treating," and the like, as used herein, refer to obtaining a desired pharmacologic and/or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter. "Treatment" can also mean prolonging survival as compared to expected survival if not receiving treatment. Those in need of treatment include those already with the condition or disorder as well as those prone to have the condition or disorder or those in which the condition or disorder is to be prevented.

The term "SARS-Co-V-2 infection", as used herein, refers not only to symptoms of the respiratory pathways (i.e. from shortness of breath to pneumonia) but also to a broad group of clinical symptoms affecting a variety of tissues and organs upon infection by SARS-CoV-2, such as organ failure, heart problems, acute respiratory distress syndrome, blood clots, acute kidney injury and additional opportunistic viral and bacterial infections.

### Immunogenic composition and vaccine of the invention

The results show that all forms of the polypeptides and oligomers of the invention elicited an immune response in the test animals, thereby inducing a remarkably strong humoral immune response against RBD. The examples demonstrate that the production of neutralizing antibodies against SARS-COV-2 can be achieved with the polypeptides and oligomers of the invention.

In a further aspect, the invention relates to an immunogenic composition or a vaccine comprising the polypeptide, the oligomer, the polynucleotide or the vector according to the invention and a pharmaceutically suitable carrier or excipient.

In one preferred embodiment, the immunogenic composition or vaccine can be used in the prevention or treatment of a disease caused by the infection of SARS-CoV-2

The immunogenic polypeptides and variants described herein are useful in the development of immunogenic compositions or vaccines used to elicit a humoral or cellular immune response. Accordingly, the polypeptides and nucleic acids of the invention find application as a prophylactic vaccine, to reduce the replication of SARS-CoV-2 in already infected patients and limit the infectivity of virus in a vaccinated patient. Such vaccines can include a polypeptide, a nucleic acid molecule encoding said polypeptides or a host cell expressing said polypeptide.

Examples of suitable adjuvants include aluminum salts. These adjuvants have been useful for some vaccines including hepatitis B, diphtheria, polio, rabies, and influenza. Other useful adjuvants include Complete Freund's Adjuvant (CFA), Incomplete Freund's Adjuvant (IFA), muramyl dipeptide (MDP), synthetic analogues of MDP, N-acetylmuramyl-L-alanyl-Disoglutamyl-L-alanine-2-[1,2-dipalmitoyl-s-glycero-3-(hydroxyl phosphoryloxy)]ethylamide (MTP-PE) and compositions containing a degradable oil and an emulsifying agent, wherein the oil and emulsifying agent are present in the form of an oil-in-water emulsion having oil droplets all of which are substantially less than one micron in diameter.

The formulation of vaccines or immunogenic compositions according to the invention will employ an effective amount of the antigen of interest. That is, it will include an amount of antigen, which in combination with the adjuvant will cause the subject to produce a specific and sufficient immunological response, so as to impart protection against a subsequent infection by SARS-CoV-2 to the subject. When used as an immunogenic composition, the formulation will contain an amount of antigenic polypeptide which, in combination with the adjuvant, will cause the subject to produce specific antibodies which may be used for diagnostic or therapeutic purposes.

The vaccine compositions of the invention may be useful for the prevention or therapy of SARS-CoV-2 infection. While all animals that can be afflicted with SARS-CoV-2 or their equivalents can be treated in this manner, the vaccines of the invention are directed particularly to their preventive and therapeutic uses in humans. Often, more than one administration may be required to bring about the desired prophylactic or therapeutic effect; the exact protocol (dosage and frequency) can be established by standard clinical procedures. In various embodiments of the invention a pharmaceutically acceptable vehicle or carrier is an adjuvant.

The invention also provides a method for stimulating the formation of antibodies capable of neutralizing an infection by a SARS-CoV-2 in at least one mammalian species. Such a method includes immunizing a subject (i.e. a mammal) with a composition containing one or more variant polypeptides according to the invention so that antibodies capable of neutralizing infection by SARS-CoV-2 are produced. Preferred polypeptides are those which can elicit antibodies which neutralize primary isolates in two or more clades. Antibody formation can be determined by obtaining a sample from the subject and evaluating whether the sample contains antibodies which specifically bind to the one or more variant polypeptides (e.g. by ELISA assays) and are capable of neutralizing one or more SARS-CoV-2 viral isolates (e.g. in a neutralization assay).

The vaccine compositions are administered in any conventional manner which will introduce the vaccine into the animal, usually by injection. For oral administration, the vaccine composition can be administered in a form similar to those used for the oral administration of other proteinaceous materials. As discussed above, the precise amounts and formulations for use in either prevention or therapy can vary depending on, for example, the circumstances of the inherent purity and activity of the antigen, any additional ingredients, carriers or the method of administration.

By way of non-limiting illustration, the vaccine dosages administered will typically be, with respect to the antigen, a minimum of about 1 µg/dose, more typically a minimum of about 0.02 mg/dose, and often a minimum of about 1 mg/dose. The maximum dosages are typically not as critical. Usually, however, the dosage will be no more than 500 mg/dose, often no more than 250 mg/dose. These dosages can be suspended in any appropriate pharmaceutical vehicle or carrier in sufficient volume to carry the dosage. Generally, the final volume, including carriers, adjuvants, and the like, typically will be at least 0.1 ml, more typically at least about 0.2 ml. The upper limit is governed by the practicality of the amount to be administered, generally no more than about 0.5 ml to about 5.0 ml.

The polypeptide variants or vectors that express said polypeptide can be administered directly into animal or human tissues by intravenous, intramuscular, intradermal, intraperitoneal, intranasal and oral inoculation routes. The specific method used to introduce the immunogen variants or their expressing vectors into the target animal or human tissue is not critical to the present invention and can be selected from previously described vaccination procedures in the art.

### Method for producing the polypeptides of the invention

The authors have also develop methods for expressing the dimer-forming and trimer-forming polypeptides of the invention using expression systems, in particular, vectors derived from insect viruses and an insect cell line. When the polypeptides are expressed from a polynucleotide which encodes the polypeptide according to the invention fused in frame to a signal sequence, the polypeptides are secreted into the cell culture media in their oligomeric (dimer or trimer) state, from which they can be purified. If the dimer-forming polypeptide contains a protease target site, the dimers can also be treated with the protease in order to remove the histidine-rich region as explained above.

Thus, in a further aspect, the invention relates to a method for producing a recombinant protein comprising a receptor binding domain (RBD) region derived from protein S of SARS-CoV-2 or a functionally equivalent and immunologically active variant thereof, comprising the steps of:
(i) expressing in suitable expression system a polynucleotide according to invention so as to produce the protein encoded by the polynucleotide and
(ii) recovering the recombinant protein from the supernatant.

"Recovering the recombinant protein" refers to any method of protein purification, for example and without limitation, chromatography, in particular, affinity chromatography.

According to the present invention, the polypeptide may be a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded. In another preferred embodiment, the polypeptide is an isolated polypeptide, wherein the term "isolated" or "recovered" means that the polypeptide has been enriched compared to its state upon production using a biotechnological or synthetic approach and is preferably pure, i.e. at least 60, 70, 80, 90, 95 or 99 percent of the polypeptide in the respective liquid consists of said polypeptide as judged by SDS polyacrylamide gel electrophoresis followed by Coomassie blue staining and visual inspection. Preferably any polypeptide on a carrier used as a means to capture an antibody is pure.

Non-limiting examples of vectors that can be used to express the polypeptides according to the invention include any of the vectors explained above in the context of the vectors according to the invention such as viral-based vectors (e.g., retrovirus, adenovirus, adeno-associated virus, lentivirus), baculovirus vectors (see, Examples), plasmid vectors, non-viral vectors, mammalians vectors, mammalian artificial chromosomes (e.g., liposomes, particulate carriers, etc.) and combinations thereof.

Suitable host cells include, but are not limited to, bacterial, mammalian, baculovirus/insect, yeast, plant and Xenopus cells. For example, a number of mammalian cell lines are known in the art and include primary cells as well as immortalized cell lines available from the American Type Culture Collection (A.T.C.C.), such as, but not limited to, MDCK, BHK, VERO, MRC-5, WI-38, HT1080, 293, 293T, RD, COS-7, CHO, Jurkat, HUT, SUPT, C8166, MOLT4/clone8, MT-2, MT-4, H9, PM1, CEM, myeloma cells (e.g., SB20 cells) and CEMX174 (such cell lines are available, for example, from the ATCC). Similarly, bacterial hosts such as E. coli, Bacillus subtilis, and Streptococcus spp., will find use with the present expression constructs. Yeast hosts useful in the present disclosure include inter alia, *Saccharomyces cerevisiae, Candida albicans, Candida maltosa, Hansenula polymorpha, Kluyveromyces fragilis, Kluyveromyces lactis, Pichia guillerimondii, Pichia pastoris, Schizosaccharomyces pombe* and *Yarrowia lipolytica.* Fungal hosts include, for example, Aspergillus.

Insect cells for use with baculovirus expression vectors include, inter alia, *Aedes aegypti, Autographa californica, Bombyx mori, Drosophila melanogaster, Spodoptera frugiperda,* and *Trichoplusia ni.*

In a preferred embodiment, the expression system is an insect or mammalian cell line.

### Method for the detection of the presence of antibodies specific for the spike protein of SARS-CoV-2 in a sample

The authors of the invention have found that the polypeptides and oligomers of the invention provide a reliable target for detection of antibodies specific for the spike protein of SARS-CoV-2 in a sample. If the sample is obtained from a subject, presence or absence of immunity against SARS-CoV-2 in said subject can be efficiently determined based on the presence or absence, respectively, of neutralizing or no-neutralizing antibodies against the S protein in the sample. The method also contemplates the detection of the presence of antibodies in samples which are not obtained from a subject, for example but not limited to, antibodies produced *in vitro* using cell lines, such as hibridomas.

Thus, in a further aspect, the invention relates to a method for the detection of the presence of antibodies specific for the SARS-CoV-2 spike protein in a sample comprising the steps of:
(i) contacting the sample with a polypeptide or with an oligomer according to invention, and
(ii) detecting the formation of a complex between antibodies specific for the SARS-CoV-2 spike protein present in the sample and the polypeptide or oligomer of step (i).

In a preferred embodiment, the detection step (ii) is carried after the complexes containing the polypeptide or oligomer and the antibodies specific for the SARS-CoV-2 spike protein are separated from the remaining components of the sample.

In a further aspect, the invention relates to a method for detecting whether a subject has suffered an infection by SARS-CoV-2 comprising the detection of antibodies against the SARS-CoV-2 spike protein in a sample of the subject by the method for detection of the presence of antibodies specific for the SARS-CoV-2 spike protein in a sample according to the invention.

In a further preferred embodiment, the polypeptide or the oligomer of the invention is immobilized.

As used herein, the term "antibody" relates to a monomeric or multimeric protein which comprises at least one polypeptide having the capacity for binding to a determined antigen, or epitope within the antigen, and comprising all or part of the light or heavy chains.

The term "antibody" also includes any type of known antibody, such as, for example, polyclonal antibodies, monoclonal antibodies and genetically engineered antibodies, such as chimeric antibodies, humanized antibodies, primatized antibodies, human antibodies and bispecific antibodies (including diabodies), multispecific antibodies (e.g. bispecific antibodies), and antibody fragments so long as they exhibit the desired biological activity.

The invention also comprises the use of fragments of the different types of antibodies mentioned above which substantially preserve the activity of the antibody. The term "antibody fragment" includes antibody fragments such as Fab, F(ab')2, Fab', single chain Fv fragments (scFv), diabodies and nanobodies.

The term "specific for" or "specifically binds" when referring to antibodies and antigen binding fragments thereof means that the antibody binds to the polypeptides and oligomers of the invention or variants thereof with no or insignificant binding to other proteins. The term however does not exclude the fact that antibodies may also be cross-reactive with other forms of the polypeptides or oligomers of the invention. Typically, the antibody binds with an association constant (Kd) of at least about 1 x 10⁻⁶ M or 10⁻⁷ M, or about 10⁻⁸ M to 10⁻⁹ M, or about 10⁻¹⁰ M to 10⁻¹¹ M or higher, and binds to the predetermined antigen with an affinity that is at least two-fold greater than its affinity for binding to a non-specific antigen (e.g., BSA, casein) other than the predetermined antigen or a closely-related antigen. The phrases "an antibody recognizing an antigen" and "an antibody specific for an antigenic protein" are used interchangeably herein with the term "an antibody which binds specifically to an antigenic protein".

The phrase "specific for" or "specifically bind(s)" or "bind(s) specifically" when referring to a polypeptide refers to a polypeptide molecule which has intermediate or high binding affinity, exclusively or predominately, to a target molecule. The phrase "specifically binds to" refers to a binding reaction that is determinative of the presence of a target protein in the presence of a heterogeneous population of proteins and other biologics. Thus, under designated assay conditions, the specified binding moieties bind preferentially to a particular target protein and do not bind in a significant amount to other components present in a test sample. Specific binding to a target protein under such conditions may require a binding moiety that is selected for its specificity for a particular target antigen. A variety of assay formats may be used to select ligands that are specifically reactive with a particular protein. For example, solid-phase ELISA immunoassays, immunoprecipitation, Biacore, and Western blot are used to identify antibodies that specifically react with the polypeptides and oligomers of the invention or their variants thereof. Typically, a specific or selective reaction will be at least twice background signal or noise and more typically more than 10 times background.

The invention also relates to a method for determining whether a subject produces antibodies as a result of the administration of a vaccine, comprising the step of detecting in a sample containing antibodies from said subject the absence or presence of an antibody directed against the polypeptides or oligomers or a variant thereof according to the invention.

The invention also relates to a method for distinguishing whether a subject produces antibodies as a result of administration of a vaccine or as a result of a previous SARS-CoV-2 infection comprising the step of detecting in a sample containing antibodies from said subject the absence or presence of an antibody directed against the polypeptides or oligomers or a variant thereof according to the invention.

In a preferred embodiment, the antibody directed against the polypeptides and oligomers of the invention is an IgA, IgG or IgM class antibody, preferably IgG or IgM, more preferably IgG.

In a preferred embodiment, any antibody is detected using a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemiluminescence immunoassays and immunofluorescence techniques.

In a preferred embodiment, the presence or absence of the antibody is monitored over at least one, preferably two, more preferably four weeks.

The invention further relates to the use of polypeptides or oligomers or a variant thereof for distinguishing whether a subject produces antibodies as a result of administration of a vaccine or as a result of a previous SARS-CoV-2 infection.

In a preferred embodiment, the polypeptides or oligomers or a variant thereof according to the invention are coated on a diagnostically useful solid carrier.

In a preferred embodiment, the carrier is selected from the group comprising a bead, a test strip, a microtiter plate, a microarray, a blot, preferably from the group comprising western blot, line blot and dot blot, a glass surface, a slide, a biochip and a membrane, and is most preferably a microtiter plate or a line blot.

In a preferred embodiment, the method is for determining the efficacy of a vaccine candidate comprising the polypeptide or oligomer or a variant thereof according to the invention.

In a preferred embodiment, the sample is a blood sample, preferably from the group comprising serum, plasma and whole blood.

The present invention centers around determining the immune status of a subject, more specifically whether the subject has been infected previously, but is now healthy, has been vaccinated or had no previous exposure to SARSCoV-2 or any component of it.

The subject is from a species that may be infected by SARS-CoV-2 and may develop an immune response against it. In a preferred embodiment, the subject is a mammal or a bird, more preferably a mammal, more preferably a mammal from the group comprising goat, rabbit, horse, mouse, rat, donkey, bat, camel, primate and human, most preferably human.

The sample from such a subject may be obtained as part of a trial using animals to check the immunogenicity or efficacy of a vaccine or it may be obtained from a human subject in a clinical trial, where the titers of antibodies are preferably monitored. In a preferred embodiment, it comprises a set of representative antibodies from the subject. In another preferred embodiment, it comprises a fraction of the sample enriched with regard to antibodies, preferably with regard to a class of antibodies, for example IgG, IgA and IgM. In a preferred embodiment, the sample is a blood sample, preferably selected from the group comprising whole blood, serum or plasma. The subject may be a laboratory animal.

Preferably antibodies of the same class against the polypeptide or oligomer or a variant thereof are detected, preferably IgA, IgM or IgG, more preferably IgG.

The inventive teachings provide a kit. Such a kit is a container that comprises specific reagents required to practice the method of the invention, in particular the diagnostically useful carrier according to the present invention, optionally in addition to one or more solutions or reagents required to practice the inventive method, preferably selected from or all from the group comprising sample dilution buffer, washing buffer and buffer comprising a means or reagent for detecting any specifically captured antibody, such as a secondary antibody which may comprise a detectable label and optionally a means for detecting the latter.

According to the present invention, a medical or diagnostic device such as the diagnostically useful carrier may be prepared by expressing a recombinant variant of comprising an affinity tag, optionally with an artificial linker which may include a protease cleavage site, in a cell such as a eukaryotic or prokaryotic cell, contacting the expressed variant with a ligand binding specifically to the affinity tag, which ligand is immobilized on a solid phase, washing the solid phase such that non-specifically bound material from the cell is removed and eluting the expressed variant from the solid phase, preferably by adding an excess of non-immobilized ligand. The variant may then be immobilized on the device. Optionally, the affinity tag may be removed by contacting the variant with a protease, preferably a protease recognizing the protease cleavage site, before the immobilization. The affinity tag may be selected from the group of tags comprising Histidine, polyhistidine, 18A, ACP, Aldehyd, Avi, BCCP, Calmodulin, Chitin binding protein, E-Tag, ELK16, FLAG, flash, poly glutamate, poly aspartate, GST, GFP, HA, Isope, maltose binding protein, myc, nus, NE, ProtA, ProtC, Tho1d4, S-Tag, SnoopTag, SpyTag, SofTag, Streptavidin, Strep-tag II, T7 Epitope Tag, TAP, TC, Thioredoxin, Ty, V5, VSV and Xpress Tag. Useful proteases include, but are not limited to TEV, Thrombin, Faktor Xa or Enteropeptidase. Suitable linkers are part of vectors, for example pET vector series (Novagen).

The method and reagents according to the present invention may also be used for screening the potency and the usefulness of an antiviral drug, preferably in a library of drug candidates.

The method and reagents according to the present invention may also be used for screening whether donated blood is contaminated with SARS-CoV-2.

The methods and reagents according to the present invention may also be used for screening whether donated blood comprises antibodies to SARS-CoV-2 and may be used to prepare a medicament used to treat a patient suffering from SARS-CoV-2 and who may benefit from the administration of such antibodies. In particular, blood comprising an antibody directed against the polypeptide or oligomer or a variant thereof according to the invention is from a patient having been exposed to SARS-CoV-2. This may have been the result of an active infection or a vaccination using the polypeptide or oligomer or a variant thereof according to the invention. In a preferred embodiment, blood from a patient may be used to treat another patient suffering from a severe SARS-CoV-2 infection or to prepare a medicament for such a treatment. In another preferred embodiment, blood from a subject having been vaccinated with a vaccine comprising the polypeptide or oligomer or a variant thereof according to the invention may be used to treat another patient suffering from a severe SARS-CoV-2 infection or to prepare a medicament for such a treatment.

The method of the present invention may not only be carried out using polypeptides or oligomers having the exact sequences referred to in this application explicitly, for example by function, name, sequence or accession number, or implicitly, but also using variants of such polypeptides.

The detection of the antibody or complex for the prognosis, diagnosis, methods or kit according to the present invention comprises the use of a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemoluminiscence immunoassays and immunofluorescence techniques. In a preferred embodiment, the complex is detected using a method selected from the group comprising immunodiffusion techniques, immunoelectrophoretic techniques, light scattering immunoassays, agglutination techniques, labeled immunoassays from the group comprising radiolabeled immunoassays, chemoluminiscence immunoassays and immunofluorescence techniques. The person skilled in the art is familiar with these methods, which are also described in the state of the art. Preferably the test format is an ELISA and a microtiter plate comprising wells is used as a diagnostically useful carrier. In a preferred embodiment, the term "presence" of an antibody, as used herein, means that the antibody is detectable using such a method, preferably a method selected from the group comprising enzyme immunoassays, more preferably colorimetric assays or chemoluminescence immunoassays, most preferably colorimetric assays based on line blot and detection using alkaline phosphatase activity as described in the examples.

Preferably the polypeptide or oligomer or a variant thereof is immobilized on a solid phase of the carrier. It may be directly immobilized on the solid phase when contacted with the sample, but a competitive assay, a capture bridge assay, an immunometric assay, a class-specific second antibody on the solid phase, a class capture assay, direct or indirect may also be used. More preferably, the solid phase is a test strip or a well of a microtiter plate for ELISA or a bead, preferably a well of a microtiter plate for ELISA.

### Method for generating antibodies comprising the use of the polypeptide according to the invention.

In a further aspect, the invention relates to a method for producing a population of antibodies against SARS-CoV-2, said method comprising the steps of:
i) administering the polypeptides or oligomers according to the invention into a subject so that an immune response against said polypeptides or oligomers is elicited in said subject and;
ii) isolating from said subject antibodies, antibody fragments and/or antibody-producing cells recognizing the polypeptides or oligomers according to the invention.

An "antibody" is used in the broadest sense and specifically covers polyclonal antibodies and antibody fragments so long as they exhibit the desired biological activity. In particular, an antibody is a protein including at least one or two, heavy (H) chain variable regions (abbreviated herein as VHC), and at least one or two light (L) chain variable regions (abbreviated herein as VLC). The VHC and VLC regions can be further subdivided into regions of hypervariability, termed "complementarity determining regions" ("CDR"), interspersed with regions that are more conserved, termed "framework regions" (FR). The extent of the framework region and CDRs has been precisely defined using either the Kabat definition or the Chothia definition. Preferably, each VHC and VLC is composed of three CDRs and four FRs, arranged from amino-terminus to carboxy-terminus in the following order: FRI, CDRI, FR2, CDR2, FR3, CDR3, FR4.

The VHC or VLC chain of the antibody can further include all or part of a heavy or light chain constant region. In one embodiment, the antibody is a tetramer of two heavy immunoglobulin chains and two light immunoglobulin chains, wherein the heavy and light immunoglobulin chains are inter-connected by, e.g., disulfide bonds. The heavy chain constant region includes three domains, CHI, CH2 and CH3. The light chain constant region is comprised of one domain, CL. The variable region of the heavy and light chains contains a binding domain that interacts with an antigen. The constant regions of the antibodies typically mediate the binding of the antibody to host tissues or factors, including various cells of the immune system (e.g., effector cells) and the first component (Clq) of the classical complement system. The term "antibody" includes intact immunoglobulins of types IgA, IgG, IgE, IgD, IgM (as well as subtypes thereof), wherein the light chains of the immunoglobulin may be of types kappa or lambda.

The term antibody, as used herein, also refers to a portion of an antibody that binds to the SARS-CoV-2 spike protein e.g., a molecule in which one or more immunoglobulin chains is not full length, but which binds to the SARS-CoV-2 spike protein. Examples of binding portions encompassed within the term antibody include (i) a Fab fragment, a monovalent fragment consisting of the VLC, VHC, CL and CHI domains; (ii) a F(ab')₂ fragment, a bivalent fragment comprising two Fab fragments linked by a disulfide bridge at the hinge region; (iii) a Fc fragment consisting of the VHC and CHI domains; (iv) a Fv fragment consisting of the VLC and VHC domains of a single arm of an antibody, (v) a dAb fragment, which consists of a VHC domain; and (vi) an isolated complementarity determining region (CDR) having sufficient framework to bind, e.g. an antigen binding portion of a variable region. An antigen binding portion of a light chain variable region and an antigen binding portion of a heavy chain variable region, e.g., the two domains of the Fv fragment, VLC and VHC, can be joined, using recombinant methods, by a synthetic linker that enables them to be made as a single protein chain in which the VLC and VHC regions pair to form monovalent molecules (known as single chain Fv (scFv). Such single chain antibodies are also encompassed within the term antibody. These are obtained using conventional techniques known to those with skill in the art, and the portions are screened for utility in the same manner as are intact antibodies.

In a first step, the method for producing antibodies according to the present invention comprises the administering the polypeptides or oligomers according to the invention into a subject so that an immune response against said polypeptides or oligomers is elicited in said subject. It will be understood that the administration step can be carried out using the polypeptides or oligomers according to the present invention as well as using cells which have been genetically modified so as to express the polypeptides or oligomers.

The polypeptides and oligomers according to the invention are used as immunogens separately or in combination, either concurrently or sequentially, in order to produce antibodies specific for the SARS-CoV-2 spike protein. Non-limiting examples of suitable adjuvants for animal/veterinary use include Freund's (complete and incomplete forms), alum (aluminium phosphate or aluminium hydroxide), saponin and its purified component Quil A.

The antibodies may be either monoclonal or polyclonal produced using standard techniques well known in the art. See Harlow E, Lane D, "Antibodies: A 25 Laboratory Manual" (Cold Spring Harbor Laboratory Press, Cold Spring Harbor, NY, USA, 1988). Methods for generating other types of antibodies, such as nanobodies, chimeric antibodies and the like, are well known in the art and they are incorporated by reference herein.

In a second step, the method for producing antibodies according to the present invention comprises isolating from said subject antibodies, antibody fragments and/or antibody-producing cells recognizing the polypeptides or oligomers according to the invention.

In one instance, the antibody produced herein is further purified to obtain preparations that are substantially homogeneous for further assays and uses. Standard protein purification methods known in the art can be employed. The following procedures are exemplary of suitable purification procedures: fractionation on immunoaffinity or ionexchange columns, ethanol precipitation, reverse phase HPLC, chromatography on silica or on a cation-exchange resin such as DEAE, chromatofocusing, SDS-PAGE, ammonium sulfate precipitation, and gel filtration using, for example, Sephadex G-75. In one embodiment, protein A immobilized on a solid phase is used for immunoaffinity purification of the antibody products of the invention.

### Further aspects of the invention

1. A polypeptide comprising:
   (i) a receptor binding domain (RBD) region derived from protein S of SARS-CoV-2 as defined in SEQ ID NO:1 or a functionally equivalent and immunologically active variant thereof, and
   (ii) a histidine-rich region.
   wherein the polypeptide does not comprise a region comprising a second receptor binding domain (RBD) derived from protein S of SARS-CoV-2 as defined in SEQ ID NO:1 or a functionally equivalent and immunologically active variant thereof.
2. The polypeptide of aspect 1 further comprising a target site for a protease between the regions (i) and (ii).
3. The polypeptide of aspects 1 or 2, wherein the histidine-rich region is located N-terminally with respect to the receptor binding domain (RBD) region or to the functionally equivalent and immunologically active variant thereof.
4. The polypeptide according to aspect 3, wherein the recombinant protein is defined in SEQ ID NO:2.
5. The polypeptide of aspect 1 or 2, wherein the histidine-rich region is located C-terminally with respect to the receptor binding domain (RBD) region or a functionally equivalent and immunologically active variant thereof.
6. The polypeptide according to aspect 5, wherein the recombinant protein is as defined in SEQ ID NO:3.
7. A dimer polypeptide which is formed by two monomer polypeptides, wherein each monomer polypeptide is as defined in any of aspects 1 to 6 and wherein the monomer polypeptides are not connected by a covalent bond between their respective distal N- or C-terminal amino acids.
8. A dimeric polypeptide, wherein each monomer polypeptide comprises a receptor binding domain (RBD) region derived from protein S of SARS-CoV-2 as defined in SEQ ID NO:1 or a functionally equivalent and immunologically active variant thereof, and wherein the monomer polypeptides are not connected by a covalent bond between their respective distal N- or C-terminal amino acids.
9. The dimer polypeptide of aspect 8 wherein in each of the monomers the histidine-rich region is located N-terminally with respect to the receptor binding domain (RBD) region or to the functionally equivalent and immunologically active variant thereof.
10. The dimer polypeptide of aspect 8 wherein in each of the monomers the histidine-rich region is located C-terminally with respect to the receptor binding domain (RBD) region or a functionally equivalent and immunologically active variant thereof.
11. A method of producing a polypeptide in form of a dimer, comprising the steps of:
   (iii) expressing in a suitable expression system a polynucleotide encoding a polypeptide comprising
      a. a receptor binding domain (RBD) region derived from protein S of SARS-CoV-2 as defined in SEQ ID NO:1 or a functionally equivalent and immunologically active variant thereof,
      b. a histidine-rich region and
      c. a protease target site between regions (a) and (b)
      wherein the expression is carried out under conditions adequate for allowing the dimerization of said polypeptides, so that dimer polypeptides are produced and;
   (iv) contacting the dimer polypeptides obtained in step (i) with a protease which is capable of recognizing and cleaving the target site found in the monomers, thereby producing dimer polypeptides devoid of the histidine-rich regions.
12. A method as defined in aspect 11 wherein the dimers are recovered after step (i) and/or after step (ii).
13. A polypeptide comprising:
   (i) a receptor binding domain (RBD) derived from protein S of SARS-CoV-2 as defined in SEQ ID NO:1 or a functionally equivalent and immunologically active variant thereof, and
   (ii) a trimerization domain.
14. The polypeptide of aspect 13 further comprising an affinity tag, preferably a histidine-rich region.
15. The polypeptide of aspects 13 or 14, wherein the trimerization domain as defined in SEQ ID NO:5.
16. The polypeptide according to aspect 15 which comprises a sequence as defined in SEQ ID NO:4.
17. A trimeric polypeptide which is formed by three monomer polypeptides, wherein each monomer polypeptide is as defined in any of aspects 13 to 16.
18. A polynucleotide encoding the polypeptide of any of aspects 1 to 6 or 13 to 16.
19. The polynucleotide according to aspect 18, wherein the polynucleotide further comprises a sequence encoding a signal peptide in the same open reading frame as the recombinant protein.
20. The polynucleotide according to aspect 19, wherein the sequence encoding a signal peptide is selected from SEQ ID NO:6 or SEQ ID NO:7.
21. The polynucleotide according to any aspect 18 to 20, wherein the polynucleotide comprises a sequence optimized for expression in insect cells.
22. The polynucleotide according to aspect 21 which comprises the sequences SEQ ID NO:8 or SEQ ID NO:9.
23. A vector comprising the polynucleotide according to any aspect 18 to 22.
24. A host cell comprising the vector according to aspect 23.
25. An polypeptide according to any of aspects 1 to 6 or 13 to 16, the oligomer according to any of aspects 7 to 10 or 17, a polynucleotide according to any of aspects 18 to 22 or a vector according to aspect 23 for use in the prevention or treatment of a disease caused by the infection by SARS-CoV-2.
26. An immunogenic composition or a vaccine comprising the polypeptide according to any of aspects 1 to 6 or 13 to 16, the oligomer according to any of aspects 7 to 10 or 17, a polynucleotide according to any of aspects 18 to 22 or a vector according to aspect 23 and a pharmaceutically suitable carrier or excipient.
27. The immunogenic composition or vaccine according to aspect 26 for use in the prevention or treatment of a disease caused by the infection by SARS-CoV-2.
28. A vaccine for use in eliciting an immune response in a subject, said vaccine comprising the polypeptide according to any of aspects 1 to 6 or 13 to 16, the oligomer according to any of aspects 7 to 10 or 17, a polynucleotide according to any of aspects 18 to 22 or a vector according to aspect 23 or the immunogenic composition or vaccine according to any one of aspects 26 or 27.
29. The vaccine for use according to aspect 28, wherein the vaccine is administered in 3 consecutive doses with 21-day intervals.
30. A method for producing a recombinant protein comprising a receptor binding domain (RBD) derived from protein S of SARS-CoV-2 or a functionally equivalent and immunologically active variant thereof, comprising the steps of:
   (i) expressing in suitable expression system a polynucleotide according to any aspect 18 to 22 so as to produce the protein encoded by the polynucleotide, and
   (ii) recovering the recombinant protein from the supernatant.
31. The method of aspect 30, wherein the expression system is an insect cell line or a mammalian cell line.
32. A method for the detection of the presence of antibodies specific for the SARS-CoV-2 spike protein in a sample comprising the steps of:
   (i) contacting the sample with polypeptide according to any of aspects 1 to 6 or 13 to 16 or with the oligomer according to any of aspects 7 or 10 or 17 and
   (ii) detecting the formation of a complex between antibodies specific for the SARS-CoV-2 spike protein present in the sample and the polypeptide or oligomer of step (i).
33. The method according to aspect 32, wherein the detection step (ii) is carried after the complexes containing the polypeptide or oligomer and the antibodies specific for the SARS-CoV-2 spike protein are separated from the remaining components of the sample.
34. The method according to aspects 32 or 33, wherein the polypeptide or the oligomer is immobilized.
35. A method for detecting whether a subject has suffered an infection by SARS-CoV-2 comprising the detection of antibodies against the SARS-CoV-2 spike protein in a sample of the subject by a method as defined in any of aspects 32 to 34.
36. A method for producing a population of antibodies against SARS-CoV-2, said method comprising the steps of:
   i) administering the polypeptide according to any of aspects 1 to 6 or 13 to 16 or the oligomer according to any of aspects 7 to 10 or 17, into a subject so that an immune response against said polypeptides or oligomers is elicited in said subject and;
   ii) isolating from said subject antibodies, antibody fragments and/or antibody-producing cells recognizing the polypeptides or oligomers according to the invention.

The invention will be described by way of the following examples which are to be considered as merely illustrative and not limitative of the scope of the invention.

### EXAMPLES

### Example 1

### Expression and purification of three forms of the RBD (Receptor Binding Domain) of the Spike protein of SARS-CoV-2 in insect cells

### Expression vectors for RBD constructs

Three synthetic genes, HIS-RBD-CNB, RBD-HIS-CNB, and RBD-FOL-CNB were designed for the expression of three versions of the RBD (residues 319-541 in HIS-RBD-CNB and RBD-HIS-CNB; residues 329-541 in RBD-FOL-CNB), of the Spike protein of the WuhanSHu-1 isolate of the SARS-CoV-2 virus (GenBank: MN908947.3).

### HIS-RBD-CNB

This recombinant gene encodes a chimeric protein formed by the signal peptide (MVSAIVLYVLLAAAAHSAFA) of the *Autographa californica* nuclear polyhedrosis virus (AcNPV) protein gp67 (UniProtKB/Swiss-Prot Accession Number P17501), followed by nine histidine residues (HHHHHHHHH) fused to the N-terminus of the RBD domain of the Spike protein (residues 319-541).

The protein sequence described was translated into DNA using the Backtranslator program (https://toolkit.tuebingen.mpg.de/tools/backtrans). The sequence was then optimized for translation in insect cells using Genscript's GenSmart^{™} Codon Optimization Tool (https://www.genscript.com). A translation termination codon (TAA) was added to the resulting sequence at its 3 'end.

The sequence of the gene is described in SEQ ID NO:8. The gene was synthesized and cloned at the multiple cloning site (between the BamHI and HindIII restriction sites) of the pFastBac1 vector (Thermo Fisher Scientific, Cat. No. 10360014) by Genscript (https://www.genscript.com/).

### RBD-HIS-CNB

This recombinant gene encodes a chimeric protein formed by the signal peptide (MFVFLVLLPLVSSQ) of the Spike protein (GenBank: MN908947.3) fused to the N-terminal end of the RBD domain of the Spike protein (residues 319-541), followed by six histidine residues at the C-terminus of RBD.

The protein sequence described was translated into DNA using the Backtranslator program (https://toolkit.tuebingen.mpg.de/tools/backtrans). The sequence was then optimized for translation in insect cells using Genscript's GenSmart^{™} Codon Optimization Tool (https://www.genscript.com). A translation termination codon (TAA) was added to the resulting sequence at its 3 'end.

The sequence of the gene is described in SEQ ID NO:9. The gene was synthesized and cloned at the multiple cloning site (between the BamHI and HindIII restriction sites) of the pFastBac1 vector (Thermo Fisher Scientific, Cat. No. 10360014) by Genscript (https://www.genscript.com).

### RBD-FOL-CNB

This recombinant chimeric protein formed by the signal peptide (MVSAIVLYVLLAAAAHSAFA) of the *Autographa californica* nuclear polyhedrosis virus (AcNPV) protein gp67 (UniProtKB/Swiss-Prot Accession Number P17501) fused to the N-terminus of the RBD domain of the Spike protein (residues 329-541), followed by the T4 foldon sequence (trimerization domain as described in SEQ ID NO:5 - UniProtKB - P10104), and nine histidine residues (HHHHHHHHH, SEQ ID NO:12) fused to the C-terminus of the foldon sequence.

The protein sequence described was translated into DNA using the Backtranslator program (https://toolkit.tuebingen.mpg.de/tools/backtrans). The sequence was then optimized for translation in insect cells using Genscript's GenSmart^{™} Codon Optimization Tool (https://www.genscript.com). A translation termination codon (TAA) was added to the resulting sequence at its 3 'end.

The sequence of the recombinant chimeric protein is described in SEQ ID NO:4.

The gene was synthesized and cloned at the multiple cloning site (between the BamHI and Hindlll restriction sites) of the pFastBac1 vector (Thermo Fisher Scientific, Cat. No. 10360014) by Genscript (https://www.genscript.com/).

### Generation of recombinant baculoviruses

The vectors described above were used to generate the corresponding recombinant baculoviruses (BV-HIS-RBD-CNB, BV-RBD-HIS-CNB, and BV-RBD-FOL-CNB). For this, the Bac-to-Bac system was used following the manufacturer's instructions (https://www.thermofisher.com)

### Expression of recombinant proteins

Recombinant baculoviruses, BV-HIS-RBD-CNB, BV-RBD-HIS-CNB, and BV-RBD-FOL-CNB, were used to infect independently insect cell cultures (bti-tn-5b1-4; Thermo Fisher Scientific) for the expression of the recombinant proteins RBD1, RBD2 and RBD 3, respectively, using a multiplicity of infection (M.O.I.) > 1/cell.

The supernatant of each set of infected cells was collected within 72 hours after infection. The medium was filtered using 0.45 µm filters and subsequently subjected to a first purification step by affinity chromatography using Nickel Agarose Extrachel^{™} columns (Agarose Bead Technologies).

The proteins retained in the columns, used to purify each of the recombinant proteins, were collected in 1 mL fractions using gradients of imidazole (0-500 mM). The collected fractions from each purification were analyzed by electrophoresis (SDS-PAGE) followed by Coomasie-blue staining. In each case, fractions containing a protein with a molecular mass similar to that expected for the corresponding recombinant polypeptide were concentrated by centrifugation on VIVASPIN 6 filters with a cut-off of 5,000 kDa (Sartorius) until reaching an approximate concentration of 3 mg/mL.

Purified proteins were subjected independently to a second purification step by gel filtration chromatography using S75 Increase columns (GE Healthcare). The collected fractions were analyzed by SDS-PAGE followed by Coomassie-blue staining. Fractions containing a protein with a molecular mass similar to that expected for the corresponding recombinant polypeptide, RBD1 (BV-HIS-RBD-CNB), RBD2 (BV-RBD-HIS-CNB) or RBD3 (BV-RBD-FOL-CNB) (≥26 and 30 kDa respectively) were concentrated by centrifugation on VIVASPIN 6 filters with a cutoff of 5,000 kDa (Sartorius) until reaching an approximate concentration of 3 mg/mL. The three purified recombinant proteins were analyzed by electrophoresis (SDS-PAGE) followed by Coomasie-blue staining.

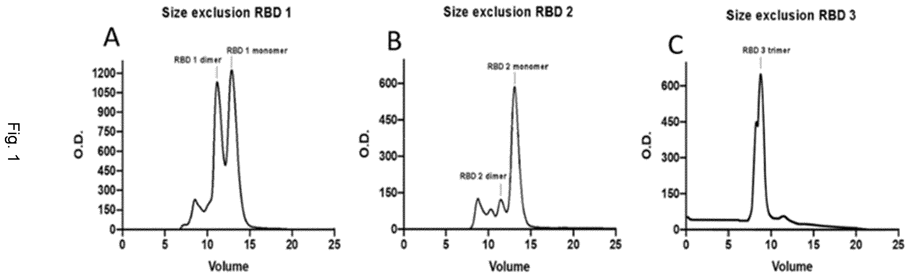

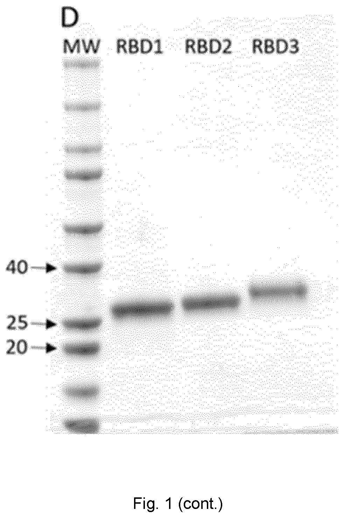

**Figure 1****.- A-C.** Chromatograms corresponding to the size exclusion elution profile of the RBD1, RBD2, and RBD3 proteins. **D.** Analysis by SDS-PAGE in reducing conditions of the different purified RBD proteins. The proteins obtained were analyzed by electrophoresis (SDS-PAGE) followed by Coomasie-blue staining. For this, 2 µg of each protein was separated by SDS-PAGE (4% -15% gradient). The proteins were then stained with Coomassie blue. Standard molecular weight markers (BioRad) are shown on the left.

The chromatographic elution profile of each of the recombinant proteins is shown in Figure 1, panels A-C. Also, the relative degree of purity of each protein can be seen on the SDS-PAGE gel under reducing conditions shown in Figure 1D. The absence of non-RBD peptides in the purified proteins, indicates a high degree of purity of each of the three purified proteins. Of note, the size exclusion chromatography conditions allow the formation of dimers and trimers, while the reducing conditions of SDS-PAGE inhibits the formation of both dimers and trimers likely due to the disruption of disulphide bonds.

As it is shown, recombinant proteins RBD1, RBD2, and RBD3, are efficiently expressed and purified from insect cells infected with the recombinant viruses described above: BV-HIS-RBD-CNB, BV-RBD-HIS-CNB, or BV-RBD-FOL-CNB, respectively (Fig.1D). As shown in Fig1 A-C, the different oligomerization of said proteins is remarkable. Thus, while RBD1 is obtained as a monomer and as a dimer in similar proportions (Fig. 1A), the RBD2 protein is obtained mostly as a monomer and to a small extent as a dimer (Fig. 1B). The RBD3 protein is produced and purified only as a trimer (Fig. 1C).

### Example 2

### Antigenic characteristics of recombinant proteins RBD1 and RBD2

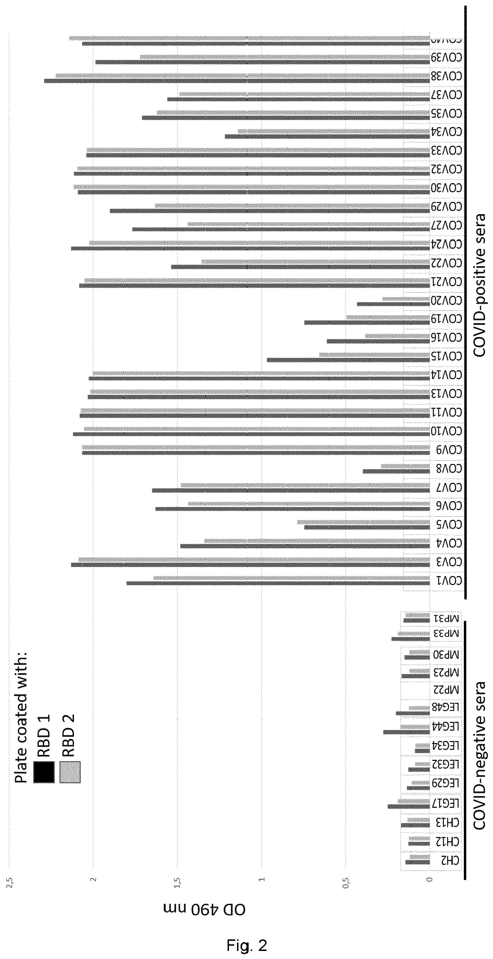

First, recombinant proteins RBD1 or RBD2, diluted in PBS at a concentration of 1 µg/mL were used for coating 96 well standard ELISA plates. After that, plates were incubated with 1:400 dilutions of 30 SARS-CoV-2 positive human serum samples and 14 pre-COVID-19 negative human serum samples. Results are shown in Figure 2 using 96 wells ELISA plates coated overnight with either RBD1 or RBD2 diluted in PBS (1 µg/mL). In brief, after coating the plates with the corresponding recombinant proteins, wells were washed three times with PBS, and later incubated with blocking solution (PBS-casein [1%], Bio Rad) at room temperature (RT) for 1 h. Then, wells were washed again, and incubated (1h at RT) with a 1:400 dilution of 30 different COVID-19 positive sera, or 14 different pre-COVID-19 negative human serum samples. After that, wells were washed 5 times, and incubated (1h at RT) with goat-anti human IgG-HRP (Abcam). After washing wells 5 times, they were incubated with OPD solution for 15 min. Then, the reaction was stopped by addition of H₂SO₄ (3M). Absorbance at 490 nm was measured in a spectrophotometer.

These results demonstrated that both RBD1 and RBD2 proteins showed similar antigenic presentation in ELISA when analysed head-to-head with a battery of anti-SARS-CoV-2 positive human sera, since both are recognized similarly (compare black and grey bars in Figure 2).

### Example 3

### Immunogenicity induced by recombinant proteins RBD1, RBD2 and RBD3

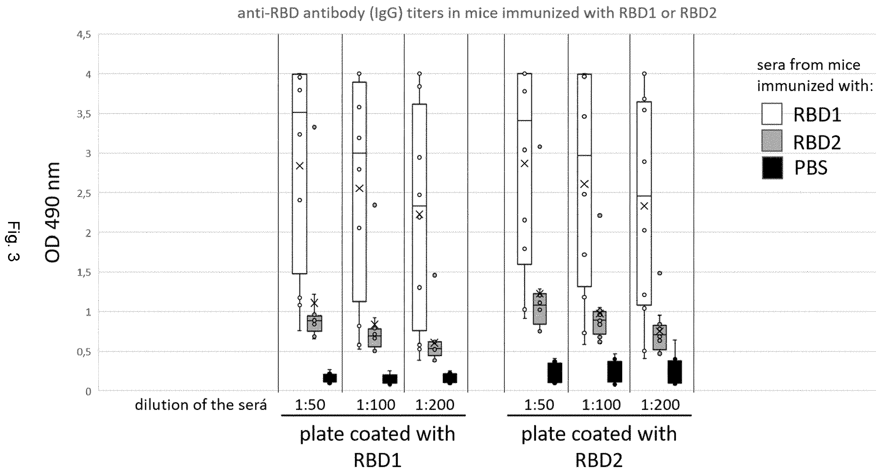

First, to study the immunogenicity of RBD1 and RBD2 recombinant proteins, groups of 10 mice (6-week-old BALB/c females) were immunized intraperitoneally (ip) with 3 doses of RBD1 protein (50 µg/mouse per dose), RBD2 protein (50 µg/mouse per dose), or with PBS (negative control). In all cases, Carbopol (1mg/mL) was used as adjuvant. Doses were administered at 21-day intervals. 21 days after the last immunization, mice were bled, and their sera were individually analyzed by ELISA for their reactivity against RBD1 or RBD2 proteins. Regardless of whether the plate was coated with RBD1 or RBD2, the results were comparable. The results are shown in Figure 3. In detail, 96 wells ELISA plates were coated overnight with either RBD1 or RBD2 diluted in PBS (1 µg/mL). After that, wells were washed three times with PBS, and later incubated with blocking solution (PBS-casein [1%], Bio Rad) at room temperature (RT) for 1 h. Then, wells were washed again, and incubated (1h at RT) with serial dilutions (1:50, 1:100 or 1:200) of sera from mice (10 per group) immunized with either RBD1, RBD2 or PBS, as described above. After that, wells were washed again 5 times, and incubated (1h at RT) with rabbit-anti mouse IgG-HRP (Abcam). After washing wells 5 times, they were incubated with OPD solution for 15 min. Then, the reaction was stopped by addition of H₂SO₄ (3M). Absorbance at 490 nm was measured in a spectrophotometer

As shown in Figure 3, and similarly to the results described in Example 2, coating ELISA plates with either RBD1 or RBD2 resulted in similar reactivity of anti-RBD mice sera. However, mice immunized with RBD1 exhibited a considerably higher anti-RBD1 or anti-RBD2 antibody titer (white bars), than animals immunized with RBD2 (grey bars). Furthermore, these differences are statistically significant (p <0.005). This unexpected result shows that in the tested conditions, RBD1 is considerably a more potent immunogen than RBD2.

Second, we decided to verify these intriguing results using more restrictive conditions: i) different route of immunization, ii) different adjuvant, iii) reducing the immunization doses, and iv) reducing the number of immunogen administration doses.

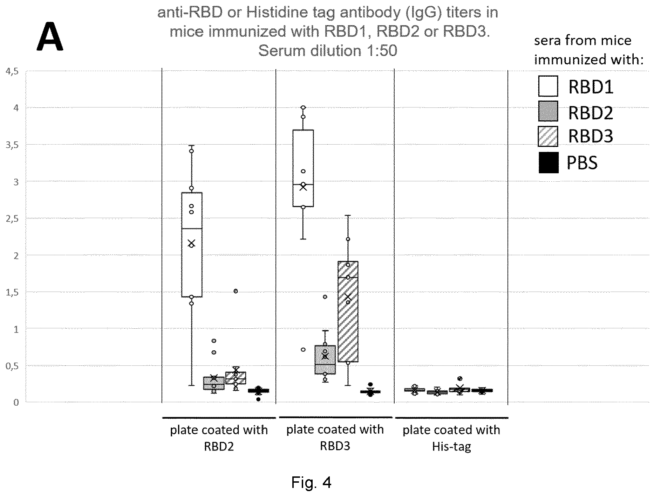

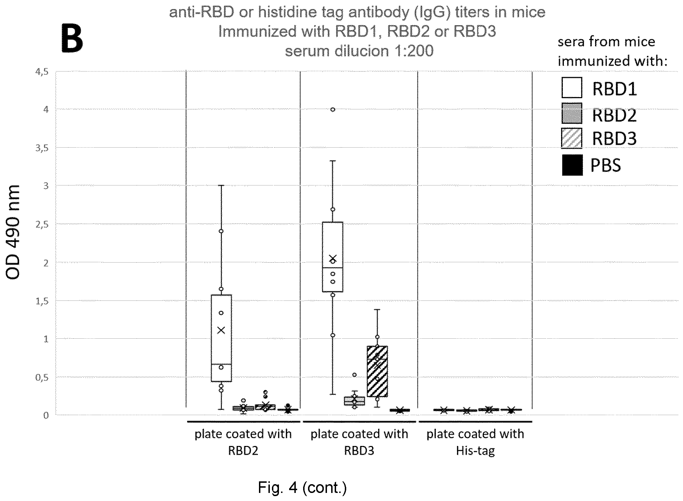

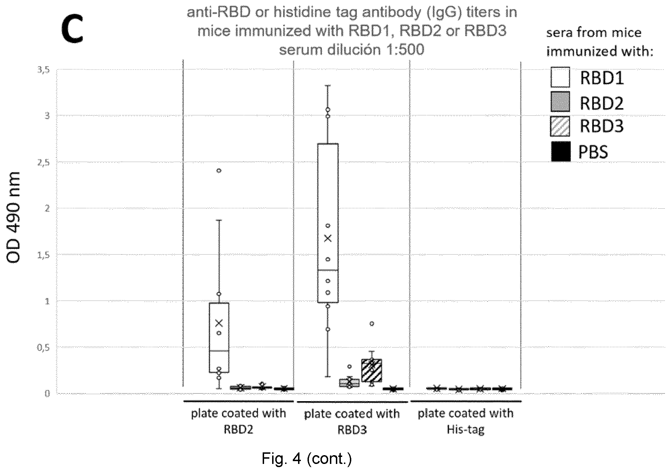

For this, groups of 10 mice (6-week-old BALB/c females) were immunized intramuscularly (im) with 2 doses of RBD1 protein (20 µg/mouse per dose), RBD2 protein (20 µg/mouse per dose), RBD3 (20 µg/mouse per dose), or with PBS (negative control). In all cases, aluminium hydroxide (Sigma-Aldrich, Cat. 239186) was used as adjuvant. Doses were administered at 21-day intervals. 21 days after the last immunization, mice were bled, and their sera were individually analyzed by ELISA for their reactivity against RBD2 (since results using RBD1 or RBD2 for coating plates were comparable we decided to use only RBD2), RBD3 or a histidine tag. Results are shown in Figure 4 A-C using 96 wells ELISA plates coated overnight with either RBD2, RBD3 or histidine tag, diluted in PBS (1 µg/mL). In brief, after coating the plates with the corresponding recombinant proteins, wells were washed three times with PBS, and later incubated with blocking solution (PBS-casein [1%], Bio Rad) at room temperature (RT) for 1 h. Then, wells were washed again, and incubated (1h at RT) with serial dilutions: 1:50 (Fig.4A), 1:200 (Fig.4B) or 1:500 (Fig.4C) of sera from mice (10 per group) immunized with either RBD1, RBD2, RBD3 or PBS, as described above. After that, wells were washed again 5 times, and incubated (1h at RT) with rabbit-anti mouse IgG-HRP (Abcam). After washing wells 5 times, they were incubated with OPD solution for 15 min. Then, reaction was stopped by addition of H₂SO₄ (3M). Absorbance at 490 nm was measured in a spectrophotometer.

As it is shown at the three dilutions tested (Figure 4 panels A-C), the results confirmed that all forms of the recombinant proteins tested elicited an immune response in the test animals, with RBD1 and RBD3 inducing a remarkably strong humoral immune response against RBD. Unexpectedly, results also showed that these differences are magnified when plates are coated with RBD3, indicating that RBD epitopes are better exposed in RBD3, at least when used for ELISA plates coating. Negligible immune responses against his-tag indicate that the reactivity of the different sera is directed against RBD epitopes. In this regard, the extremely low responses in animal immunized with PBS, indicate the specificity of the data.

### Example 4

### Neutralization of SARS-CoV-2 infectivity by the serum of mice immunized with RBD1 protein

Based on the results shown in Example 3, it was of relevance to demonstrate whether the sera of the mice immunized with the RBD1 protein were capable of neutralizing the infectivity of the SARS-CoV-2 *in vitro.*

For this, a seroneutralization test was carried out. In this assay, dilutions of virus containing 100 plaque-forming units (PFUs) of SARS-CoV-2 were incubated for 1 h at 37°C with 1:3 serial dilutions of: i) the sera of two mice immunized with the RBD1 protein, ii) the serum of a mouse immunized with PBS (negative control), or iii) a commercial monoclonal antibody (Sino Biological, Cat. No. 40592-MM57), with a demonstrated neutralizing activity, as a positive control (650 µg/mL stock solution). Then, the serum/virus mixtures were added in duplicate over confluent monolayers of Vero E6 cells grown in 12-well plates. After 1 h of adsorption the inoculum was removed and a mixture of DMEM-2% FCS with agarose (0.6%) was added. At 3 days post-infection, cells were fixed with 10% formaldehyde, the medium was removed, the cells were stained with crystal violet, and viral plaques were counted. The reduction in the number of lysis plaques with respect to the negative control indicates the degree of neutralization of the serum. The results, shown in Table I, demonstrate that immunization with the RBD1 recombinant protein induces anti-RBD antibodies with a neutralizing capacity against SARS-CoV-2 comparable to 650 µg/mL of purified monoclonal antibody 40592-MM57.

**Table I**

| | **% NEUTRALIZATION** | | | |
|---|---|---|---|---|
| **DILUTION** | **CONTROL +** | **MOUSE 1** | **MOUSE 2** | **CONTROL-** |
| 1:8 | 100 | 100 | 100 | 0 |
| 1:24 | 89 | 83 | 98 | 0 |
| 1:72 | 71 | 69 | 65 | 0 |
| 1:216 | 33 | 38 | 27 | 0 |
| 1:648 | 17 | 25 | 0 | 0 |

## Claims

1. A polypeptide comprising:
(i) a receptor binding domain (RBD) region derived from protein S of SARS-CoV-2 as defined in SEQ ID NO:1 or a functionally equivalent and immunologically active variant thereof, and
(ii) a histidine-rich region.
wherein the polypeptide does not comprise a region comprising a second receptor binding domain (RBD) region derived from protein S of SARS-CoV-2 as defined in SEQ ID NO:1 or a functionally equivalent and immunologically active variant thereof.

2. The polypeptide of claim 1 further comprising a target site for a protease between the regions (i) and (ii).

3. The polypeptide of claims 1 or 2, wherein the histidine-rich region is located N-terminally with respect to a receptor binding domain (RBD) region or to the functionally equivalent and immunologically active variant thereof.

4. The polypeptide of claim 1 or 2, wherein the histidine-rich region is located C-terminally with respect to a receptor binding domain (RBD) region or a functionally equivalent and immunologically active variant thereof.

5. A dimer polypeptide which is formed by two monomer polypeptides, wherein each monomer polypeptide is as defined in any of claims 1 to 4 and wherein the monomer polypeptides are not connected by a covalent bond between their respective distal N- or C-terminal amino acids.

6. A dimeric polypeptide, wherein each monomer polypeptide comprises a receptor binding domain (RBD) region derived from protein S of SARS-CoV-2 as defined in SEQ ID NO:1 or a functionally equivalent and immunologically active variant thereof, and wherein the monomer polypeptides are not connected by a covalent bond between their respective distal N- or C-terminal amino acids.

7. The dimer polypeptide of claim 6 wherein in each of the monomers the histidine-rich region is located N-terminally with respect to the receptor binding domain (RBD) region or to the functionally equivalent and immunologically active variant thereof.

8. The dimer polypeptide of claim 6 wherein in each of the monomers the histidine-rich region is located C-terminally with respect to the receptor binding domain (RBD) region or a functionally equivalent and immunologically active variant thereof.

9. A polypeptide comprising:
(i) a receptor binding domain (RBD) derived from protein S of SARS-CoV-2 as defined in SEQ ID NO:1 or a functionally equivalent and immunologically active variant thereof, and
(ii) a trimerization domain.

10. A trimeric polypeptide which is formed by three monomer polypeptides, wherein each monomer polypeptide is as defined in any of claims 1 to 4 or 9.

11. A polynucleotide encoding the polypeptide of any of claims 1 to 4 or 9.

12. An polypeptide according to any of claims 1 to 4 or 9, the oligomer according to any of claims 5 to 8 or 10, a polynucleotide according to claim 11 for use in the prevention or treatment of a disease caused by the infection by SARS-CoV-2.

13. An immunogenic composition or a vaccine comprising the polypeptide according to any of claims 1 to 4 or 9, the oligomer according to any of claims 5 to 8 or 10, a polynucleotide according to claim 11 and a pharmaceutically suitable carrier or excipient.

14. A vaccine for use in eliciting an immune response in a subject, said vaccine comprising the polypeptide according to any of claims 1 to 4 or 9, the oligomer according to any of claims 5 to 8 or 10, a polynucleotide according to claim 11 or the immunogenic composition or vaccine according to claim 13.

15. A method for the detection of the presence of antibodies specific for the SARS-CoV-2 spike protein in a sample comprising the steps of:
(i) contacting the sample with polypeptide according to any of claims 1 to 4 or 9, or with the oligomer according to any of claims 5 to 8 or 10, and
(ii) detecting the formation of a complex between antibodies specific for the SARS-CoV-2 spike protein present in the sample and the polypeptide or oligomer of step (i).


SEQUENCE LISTING

<110>  CONSEJO SUPERIOR DE INVESTIGACIONES CIENTÍFICAS

<120>  IMMUNOGENIC POLYPEPTIDES AND USES THEREOF

<130>  P20393EP00

<160>  19

<170>  PatentIn version 3.5

<210>  1
<211>  223
<212>  PRT
<213>  SARS-CoV2

<400>  1

Arg Val Gln Pro Thr Glu Ser Ile Val Arg Phe Pro Asn Ile Thr Asn
1               5                   10                  15

Leu Cys Pro Phe Gly Glu Val Phe Asn Ala Thr Arg Phe Ala Ser Val
            20                  25                  30

Tyr Ala Trp Asn Arg Lys Arg Ile Ser Asn Cys Val Ala Asp Tyr Ser
        35                  40                  45

Val Leu Tyr Asn Ser Ala Ser Phe Ser Thr Phe Lys Cys Tyr Gly Val
    50                  55                  60

Ser Pro Thr Lys Leu Asn Asp Leu Cys Phe Thr Asn Val Tyr Ala Asp
65                  70                  75                  80

Ser Phe Val Ile Arg Gly Asp Glu Val Arg Gln Ile Ala Pro Gly Gln
                85                  90                  95

Thr Gly Lys Ile Ala Asp Tyr Asn Tyr Lys Leu Pro Asp Asp Phe Thr
            100                 105                 110

Gly Cys Val Ile Ala Trp Asn Ser Asn Asn Leu Asp Ser Lys Val Gly
        115                 120                 125

Gly Asn Tyr Asn Tyr Leu Tyr Arg Leu Phe Arg Lys Ser Asn Leu Lys
    130                 135                 140

Pro Phe Glu Arg Asp Ile Ser Thr Glu Ile Tyr Gln Ala Gly Ser Thr
145                 150                 155                 160

Pro Cys Asn Gly Val Glu Gly Phe Asn Cys Tyr Phe Pro Leu Gln Ser
                165                 170                 175

Tyr Gly Phe Gln Pro Thr Asn Gly Val Gly Tyr Gln Pro Tyr Arg Val
            180                 185                 190

Val Val Leu Ser Phe Glu Leu Leu His Ala Pro Ala Thr Val Cys Gly
        195                 200                 205

Pro Lys Lys Ser Thr Asn Leu Val Lys Asn Lys Cys Val Asn Phe
    210                 215                 220

<210>  2
<211>  233
<212>  PRT
<213>  Artificial Sequence

<220>
<223>  x9His-RBD SARS-CoV2

<400>  2

Ala His His His His His His His His His Arg Val Gln Pro Thr Glu
1               5                   10                  15

Ser Ile Val Arg Phe Pro Asn Ile Thr Asn Leu Cys Pro Phe Gly Glu
            20                  25                  30

Val Phe Asn Ala Thr Arg Phe Ala Ser Val Tyr Ala Trp Asn Arg Lys
        35                  40                  45

Arg Ile Ser Asn Cys Val Ala Asp Tyr Ser Val Leu Tyr Asn Ser Ala
    50                  55                  60

Ser Phe Ser Thr Phe Lys Cys Tyr Gly Val Ser Pro Thr Lys Leu Asn
65                  70                  75                  80

Asp Leu Cys Phe Thr Asn Val Tyr Ala Asp Ser Phe Val Ile Arg Gly
                85                  90                  95

Asp Glu Val Arg Gln Ile Ala Pro Gly Gln Thr Gly Lys Ile Ala Asp
            100                 105                 110

Tyr Asn Tyr Lys Leu Pro Asp Asp Phe Thr Gly Cys Val Ile Ala Trp
        115                 120                 125

Asn Ser Asn Asn Leu Asp Ser Lys Val Gly Gly Asn Tyr Asn Tyr Leu
    130                 135                 140

Tyr Arg Leu Phe Arg Lys Ser Asn Leu Lys Pro Phe Glu Arg Asp Ile
145                 150                 155                 160

Ser Thr Glu Ile Tyr Gln Ala Gly Ser Thr Pro Cys Asn Gly Val Glu

165                 170                 175

Gly Phe Asn Cys Tyr Phe Pro Leu Gln Ser Tyr Gly Phe Gln Pro Thr
            180                 185                 190

Asn Gly Val Gly Tyr Gln Pro Tyr Arg Val Val Val Leu Ser Phe Glu
        195                 200                 205

Leu Leu His Ala Pro Ala Thr Val Cys Gly Pro Lys Lys Ser Thr Asn
    210                 215                 220

Leu Val Lys Asn Lys Cys Val Asn Phe
225                 230

<210>  3
<211>  229
<212>  PRT
<213>  Artificial Sequence

<220>
<223>  RBD SARS-CoV2-6xHis

<400>  3

Arg Val Gln Pro Thr Glu Ser Ile Val Arg Phe Pro Asn Ile Thr Asn
1               5                   10                  15

Leu Cys Pro Phe Gly Glu Val Phe Asn Ala Thr Arg Phe Ala Ser Val
            20                  25                  30

Tyr Ala Trp Asn Arg Lys Arg Ile Ser Asn Cys Val Ala Asp Tyr Ser
        35                  40                  45

Val Leu Tyr Asn Ser Ala Ser Phe Ser Thr Phe Lys Cys Tyr Gly Val
    50                  55                  60

Ser Pro Thr Lys Leu Asn Asp Leu Cys Phe Thr Asn Val Tyr Ala Asp
65                  70                  75                  80

Ser Phe Val Ile Arg Gly Asp Glu Val Arg Gln Ile Ala Pro Gly Gln
                85                  90                  95

Thr Gly Lys Ile Ala Asp Tyr Asn Tyr Lys Leu Pro Asp Asp Phe Thr
            100                 105                 110

Gly Cys Val Ile Ala Trp Asn Ser Asn Asn Leu Asp Ser Lys Val Gly
        115                 120                 125

Gly Asn Tyr Asn Tyr Leu Tyr Arg Leu Phe Arg Lys Ser Asn Leu Lys
    130                 135                 140

Pro Phe Glu Arg Asp Ile Ser Thr Glu Ile Tyr Gln Ala Gly Ser Thr
145                 150                 155                 160

Pro Cys Asn Gly Val Glu Gly Phe Asn Cys Tyr Phe Pro Leu Gln Ser
                165                 170                 175

Tyr Gly Phe Gln Pro Thr Asn Gly Val Gly Tyr Gln Pro Tyr Arg Val
            180                 185                 190

Val Val Leu Ser Phe Glu Leu Leu His Ala Pro Ala Thr Val Cys Gly
        195                 200                 205

Pro Lys Lys Ser Thr Asn Leu Val Lys Asn Lys Cys Val Asn Phe His
    210                 215                 220

His His His His His
225

<210>  4
<211>  239
<212>  PRT
<213>  Artificial Sequence

<220>
<223>  RBD SARS-CoV2-Foldon T4

<400>  4

Phe Pro Asn Ile Thr Asn Leu Cys Pro Phe Gly Glu Val Phe Asn Ala
1               5                   10                  15

Thr Arg Phe Ala Ser Val Tyr Ala Trp Asn Arg Lys Arg Ile Ser Asn
            20                  25                  30

Cys Val Ala Asp Tyr Ser Val Leu Tyr Asn Ser Ala Ser Phe Ser Thr
        35                  40                  45

Phe Lys Cys Tyr Gly Val Ser Pro Thr Lys Leu Asn Asp Leu Cys Phe
    50                  55                  60

Thr Asn Val Tyr Ala Asp Ser Phe Val Ile Arg Gly Asp Glu Val Arg
65                  70                  75                  80

Gln Ile Ala Pro Gly Gln Thr Gly Lys Ile Ala Asp Tyr Asn Tyr Lys
                85                  90                  95

Leu Pro Asp Asp Phe Thr Gly Cys Val Ile Ala Trp Asn Ser Asn Asn
            100                 105                 110

Leu Asp Ser Lys Val Gly Gly Asn Tyr Asn Tyr Leu Tyr Arg Leu Phe
        115                 120                 125

Arg Lys Ser Asn Leu Lys Pro Phe Glu Arg Asp Ile Ser Thr Glu Ile
    130                 135                 140

Tyr Gln Ala Gly Ser Thr Pro Cys Asn Gly Val Glu Gly Phe Asn Cys
145                 150                 155                 160

Tyr Phe Pro Leu Gln Ser Tyr Gly Phe Gln Pro Thr Asn Gly Val Gly
                165                 170                 175

Tyr Gln Pro Tyr Arg Val Val Val Leu Ser Phe Glu Leu Leu His Ala
            180                 185                 190

Pro Ala Thr Val Cys Gly Pro Lys Lys Ser Thr Asn Leu Val Pro Arg
        195                 200                 205

Gly Ser Gly Tyr Ile Pro Glu Ala Pro Arg Asp Gly Gln Ala Tyr Val
    210                 215                 220

Arg Lys Asp Gly Glu Trp Val Phe Leu Ser Thr Phe Leu Ser Pro
225                 230                 235

<210>  5
<211>  29
<212>  PRT
<213>  T4 bacteriophage

<400>  5

Gly Tyr Ile Pro Glu Ala Pro Arg Asp Gly Gln Ala Tyr Val Arg Lys
1               5                   10                  15

Asp Gly Glu Trp Val Phe Leu Ser Thr Phe Leu Ser Pro
            20                  25

<210>  6
<211>  60
<212>  DNA
<213>  Autographa californica nuclear polyhedrosis virus

<400>  6
atggtaagcg ctattgtttt atatgtgctt ttggcggcgg cggcgcattc tgccttgcgc        60

<210>  7
<211>  42
<212>  DNA
<213>  SARS-CoV2

<400>  7

atgttcgtct tcctcgttct cctcccgttg gtgtccagcc ag                42

<210>  8
<211>  759
<212>  DNA
<213>  Artificial Sequence

<220>
<223>  DNA encoding signal peptide(gp67)-9xHis-amino acids 319 to 541
       (RBD) of SARS-CoV2 protein S

<400>  8
atggtaagcg ctattgtttt atatgtgctt ttggcggcgg cggcgcattc tgcctttgcg      60

catcatcatc accatcacca tcatcaccgt gtccagccca ctgagtccat tgtccgtttc     120

cctaacatca ctaacctgtg ccctttcgga gaggtgttca acgctaccag gttcgccagc     180

gtctacgctt ggaaccgcaa gcgtatcagc aactgcgtcg ccgactactc tgtgctgtac     240

aactccgcta gcttctctac tttcaagtgc tacggcgtga gccctaccaa gctgaacgac     300

ctgtgcttca ctaacgtcta cgccgactct ttcgtgatcc gcggagacga agtccgtcag     360

atcgccccag gccagaccgg aaagatcgct gactacaact acaagctgcc tgacgacttc     420

actggctgcg tgatcgcttg gaactcaaac aacctggact ccaaggtcgg tggcaactac     480

aactacctgt acaggctgtt cagaaagtct aacctgaagc ccttcgagag ggacatctca     540

accgaaatct accaggccgg ttccactcca tgcaacggtg tggagggctt caactgctac     600

ttcccactgc agtcatacgg tttccagcct accaacggag tcggttacca gccctacaga     660

gtggtcgtgc tgtccttcga actgctgcac gctcctgcca ctgtctgcgg tcccaagaaa     720

tctaccaacc tcgtcaagaa caaatgtgtc aacttctaa                           759

<210>  9
<211>  732
<212>  DNA
<213>  Artificial Sequence

<220>
<223>  DNA encoding signal peptide(Spike)-RBD SARS-CoV2-6xHis (protein)

<400>  9
atgttcgtct tcctcgttct cctcccgttg gtgtccagcc agcgtgtcca gcccactgag      60

tccattgtcc gtttccctaa catcactaac ctgtgccctt tcggagaggt gttcaacgct     120

accaggttcg ccagcgtcta cgcttggaac cgcaagcgta tcagcaactg cgtcgccgac     180

tactctgtgc tgtacaactc cgctagcttc tctactttca agtgctacgg cgtgagccct     240

accaagctga acgacctgtg cttcactaac gtctacgccg actctttcgt gatccgcgga     300

gacgaagtcc gtcagatcgc cccaggccag accggaaaga tcgctgacta caactacaag     360

ctgcctgacg acttcactgg ctgcgtgatc gcttggaact caaacaacct ggactccaag     420

gtcggtggca actacaacta cctgtacagg ctgttcagaa agtctaacct gaagcccttc      480

gagagggaca tctcaaccga aatctaccag gccggttcca ctccatgcaa cggtgtggag      540

ggcttcaact gctacttccc actgcagtca tacggtttcc agcctaccaa cggagtcggt      600

taccagccct acagagtggt cgtgctgtcc ttcgaactgc tgcacgctcc tgccactgtc      660

tgcggtccca agaaatctac caacctcgtc aagaacaaat gtgtcaactt ccatcatcat      720

caccatcact aa                                                          732

<210>  10
<211>  20
<212>  PRT
<213>  Autographa californica nuclear polyhedrosis virus

<400>  10

Met Val Ser Ala Ile Val Leu Tyr Val Leu Leu Ala Ala Ala Ala His
1               5                   10                  15

Ser Ala Phe Ala
            20

<210>  11
<211>  14
<212>  PRT
<213>  SARS-CoV2

<400>  11

Met Phe Val Phe Leu Val Leu Leu Pro Leu Val Ser Ser Gln
1               5                   10

<210>  12
<211>  9
<212>  PRT
<213>  Artificial Sequence

<220>
<223>  9x Histidines

<400>  12

His His His His His His His His His
1               5

<210>  13
<211>  6
<212>  PRT
<213>  Artificial Sequence

<220>
<223>  Thrombin cleavage site

<400>  13

Leu Val Pro Arg Gly Ser
1               5

<210>  14
<211>  7
<212>  PRT
<213>  Artificial Sequence

<220>
<223>  TEV cleavage site

<400>  14

Glu Asn Leu Tyr Phe Gln Gly
1               5

<210>  15
<211>  7
<212>  PRT
<213>  Artificial Sequence

<220>
<223>  TEV cleavage site

<400>  15

Glu Asn Leu Tyr Phe Gln Ser
1               5

<210>  16
<211>  4
<212>  PRT
<213>  Artificial Sequence

<220>
<223>  Factor Xa cleavage site

<400>  16

Ile Glu Gly Arg
1

<210>  17
<211>  4
<212>  PRT
<213>  Artificial Sequence

<220>
<223>  Factor Xa cleavage site

<400>  17

Ile Asp Gly Arg
1

<210>  18
<211>  8
<212>  PRT

<213>  Artificial Sequence

<220>
<223>  HRV 3C Protease cleavage site

<400>  18

Leu Glu Val Leu Phe Gln Gly Pro
1               5


<210>  19
<211>  210
<212>  PRT
<213>  Artificial Sequence

<220>
<223>  positions 329-541 of RBD SARS-CoV2 (last 5 residues of native
       sequence substituted with PRGS)

<400>  19

Phe Pro Asn Ile Thr Asn Leu Cys Pro Phe Gly Glu Val Phe Asn Ala
1               5                   10                  15

Thr Arg Phe Ala Ser Val Tyr Ala Trp Asn Arg Lys Arg Ile Ser Asn
            20                  25                  30

Cys Val Ala Asp Tyr Ser Val Leu Tyr Asn Ser Ala Ser Phe Ser Thr
        35                  40                  45

Phe Lys Cys Tyr Gly Val Ser Pro Thr Lys Leu Asn Asp Leu Cys Phe
    50                  55                  60

Thr Asn Val Tyr Ala Asp Ser Phe Val Ile Arg Gly Asp Glu Val Arg
65                  70                  75                  80

Gln Ile Ala Pro Gly Gln Thr Gly Lys Ile Ala Asp Tyr Asn Tyr Lys
                85                  90                  95

Leu Pro Asp Asp Phe Thr Gly Cys Val Ile Ala Trp Asn Ser Asn Asn
            100                 105                 110

Leu Asp Ser Lys Val Gly Gly Asn Tyr Asn Tyr Leu Tyr Arg Leu Phe
        115                 120                 125

Arg Lys Ser Asn Leu Lys Pro Phe Glu Arg Asp Ile Ser Thr Glu Ile
    130                 135                 140

Tyr Gln Ala Gly Ser Thr Pro Cys Asn Gly Val Glu Gly Phe Asn Cys
145                 150                 155                 160

Tyr Phe Pro Leu Gln Ser Tyr Gly Phe Gln Pro Thr Asn Gly Val Gly

                165                 170                 175

Tyr Gln Pro Tyr Arg Val Val Val Leu Ser Phe Glu Leu Leu His Ala
            180                 185                 190

Pro Ala Thr Val Cys Gly Pro Lys Lys Ser Thr Asn Leu Val Pro Arg
        195                 200                 205

Gly Ser
    210